(19)

Europäisches
Patentamt
European
Patent Office
Office européen
des brevets

(11) **EP 4 641 891 A1**

(12) **EUROPEAN PATENT APPLICATION**
published in accordance with Art. 153(4) EPC

(43) Date of publication:
29.10.2025 Bulletin 2025/44

(21) Application number: 23911918.3

(22) Date of filing: 21.12.2023

(51) International Patent Classification (IPC):
*H02K 7/09* (2006.01)      *A61B 6/03* (2006.01)
*F16C 32/04* (2006.01)     *H02K 21/16* (2006.01)

(52) Cooperative Patent Classification (CPC):
A61B 6/032; A61B 6/035; A61B 6/4275

(86) International application number:
PCT/JP2023/045875

(87) International publication number:
WO 2024/143138 (04.07.2024 Gazette 2024/27)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 27.12.2022 JP 2022210208

(71) Applicant: Canon Kabushiki Kaisha
Tokyo, 146-8501 (JP)

(72) Inventors:
• SUZUKI, Hitoshi
  Tokyo 146-8501 (JP)
• NARAHARA, Hidetada
  Tokyo 146-8501 (JP)

(74) Representative: WESER & Kollegen
Patentanwälte PartmbB
Radeckestraße 43
81245 München (DE)

(54) **ROTATION DRIVE DEVICE, CONTROL METHOD FOR SAME, AND ROTATION APPARATUS**

(57) A rotation drive apparatus and rotation equipment capable of levitating the rotor and performing rotation control of the rotor while avoiding enlargement of the whole apparatus are provided. The rotation drive apparatus includes: a rotor that includes a plurality of magnets and is rotatable in a rotation direction with a rotation axis intersecting a direction of gravity; and a stator that includes a plurality of coils, wherein the plurality of magnets includes a magnet group arranged circumferentially along the rotation direction on an outer peripheral side surface of the rotor, wherein the magnet group includes a first magnet line with different magnetic poles aligned in the rotation direction and a second magnet line with different magnetic poles aligned in a direction intersecting the rotation direction, and wherein the plurality of coils includes, above the rotor, a coil group that is arranged in an arc shape along the rotation direction and can face the magnet group.

FIG. 1A

EP 4 641 891 A1

## Description

[Technical Field]

The present invention relates to a rotation drive apparatus and a control method thereof, and rotation equipment.

[Background Art]

Gantries for computed tomography (CT), for example, are known as rotating equipment with a configuration in which a rotor is rotated in a contactless state by the action of magnetism. Furthermore, such a configuration is known to be applied to a magnetic levitation motor.

In a general X-ray CT apparatus, an X-ray tube is attached to the rotor side of a rotation unit that includes a stator and a rotor, and scanning is executed by rotating the rotor. The rotor is coupled to the annular stator via ball bearings and is rotated by a DD (Direct Drive) motor. In the annular rotor, an X-ray tube and an X-ray detector are arranged facing each other across the center of the rotor. When a subject is sent to the inside of the rotor through the entrance, an X-ray is emitted from the X-ray tube and the scan is performed while rotating the rotor.

However, in such a rotation unit of the X-ray CT apparatus, since the bearings connecting the stator and the rotor is composed of a ball bearing, the rotational speed of the rotor is limited. In recent years, in order to solve this problem, studies have been conducted to raise the rotational speed by levitating the rotor and eliminating sliding portions. As one of the levitation methods, there is a magnetic levitation method, and an apparatus for levitation control along the gravity direction of the rotor is known (see PTL 1).

[Citation List]

[Patent Literature]

PTL 1: Japanese Patent Laid-Open No. 2014-180578

[Summary of Invention]

[Technical Problem]

However, the apparatus described in PTL 1 includes a lifting actuator, an axial actuator and a radial actuator for levitating the rotor, and has a large number of actuators. The apparatus described in PTL 1 also requires additional actuators for controlling axial rotation. Thus, in performing rotation control while performing levitation control of the rotor, there is a problem that as the number of actuators increases as in the apparatus described in PTL 1, the whole apparatus becomes larger.

It is an object of the present invention to provide a rotation drive apparatus capable of levitating the rotor and performing rotation control of the rotor while avoiding enlargement of the whole apparatus, a control method thereof, and rotation equipment.

[Solution to Problem]

According to one aspect of the present invention, there is provided a rotation drive apparatus including: a rotor that includes a plurality of magnets and is rotatable in a rotation direction with a rotation axis intersecting a direction of gravity; and a stator that includes a plurality of coils, wherein the plurality of magnets includes a magnet group arranged circumferentially along the rotation direction on an outer peripheral side surface of the rotor, wherein the magnet group includes a first magnet line with different magnetic poles aligned in the rotation direction and a second magnet line with different magnetic poles aligned in a direction intersecting the rotation direction, and wherein the plurality of coils includes, above the rotor, a coil group that is arranged in an arc shape along the rotation direction and can face the magnet group.

According to another aspect of the present invention, there is provided a rotation drive apparatus including: a rotor that includes a plurality of magnets and is rotatable in a rotation direction with a rotation axis intersecting a direction of gravity; and a stator that includes a plurality of coils, wherein the plurality of magnets includes a first magnet group arranged circumferentially along the rotation direction at one end face of the rotor in a direction along the rotation axis and a second magnet group arranged circumferentially along the rotation direction at the other end face of the rotor in a direction along the rotation axis, wherein the first magnet group and the second magnet group each include a first magnet line with different magnetic poles aligned in the rotation direction and a second magnet line with different magnetic poles aligned in a direction intersecting the rotation direction, and wherein the plurality of coils includes a first coil group that is arranged circumferentially along the rotation direction and can face the first magnet group, and a second coil group that is arranged circumferentially along the rotation direction and can face the second magnet group.

According to a further aspect of the present invention, there is provided a method of controlling a rotation drive apparatus including: a rotor that includes a plurality of magnets and is rotatable in a rotation direction with a rotation axis intersecting a direction of gravity; and a stator that includes a plurality of coils, wherein the plurality of magnets includes a magnet group arranged circumferentially along the rotation direction on an outer peripheral side surface of the rotor, wherein the magnet group includes a first magnet line with different magnetic poles aligned in the rotation direction and a second magnet line with different magnetic poles aligned in a direction intersecting the rotation direction, and wherein the plurality of coils includes, above the rotor, a coil group that is arranged in an arc shape along the rotation direction and can face the magnet group, the method including controlling drive of the rotor by controlling currents applied to the plurality of coils.

According to a further aspect of the present invention, there is provided a method of controlling a rotation drive apparatus including: a rotor that includes a plurality of magnets and is rotatable in a rotation direction with a rotation axis intersecting a direction of gravity; and a stator that includes a plurality of coils, wherein the plurality of magnets includes a first magnet group arranged circumferentially along the rotation direction at one end face of the rotor in a direction along the rotation axis and a second magnet group arranged circumferentially along the rotation direction at the other end face of the rotor in a direction along the rotation axis, wherein the first magnet group and the second magnet group each include a first magnet line with different magnetic poles aligned in the rotation direction and a second magnet line with different magnetic poles aligned in a direction intersecting the rotation direction, and wherein the plurality of coils includes a first coil group that is arranged circumferentially along the rotation direction and can face the first magnet group, and a second coil group that is arranged circumferentially along the rotation direction and can face the second magnet group, the method including controlling drive of the rotor by controlling currents applied to the plurality of coils.

[Advantageous Effects of Invention]

According to the present invention, rotation control of the rotor can be performed by levitating the rotor while avoiding enlargement of the whole apparatus.

[Brief Description of Drawings]

[FIG. 1A]

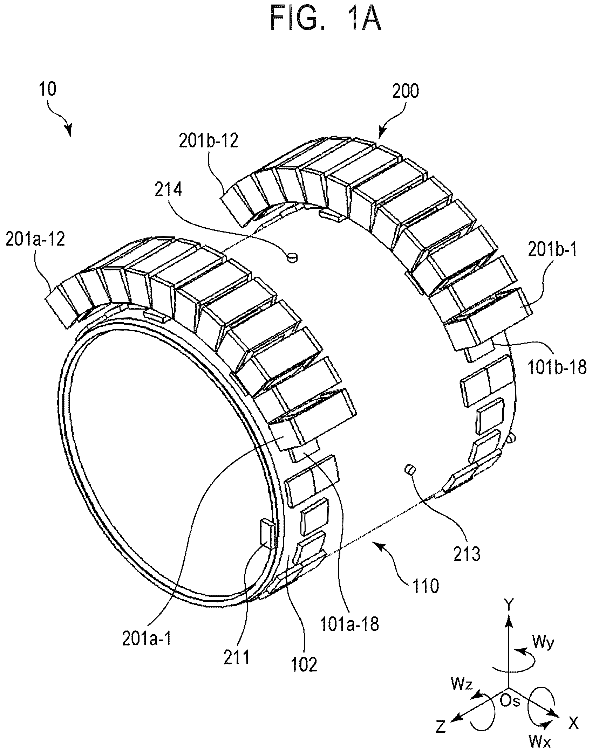

FIG. 1A is a schematic diagram illustrating a rotation drive apparatus according to a first embodiment of the present invention.
[FIG. 1B]

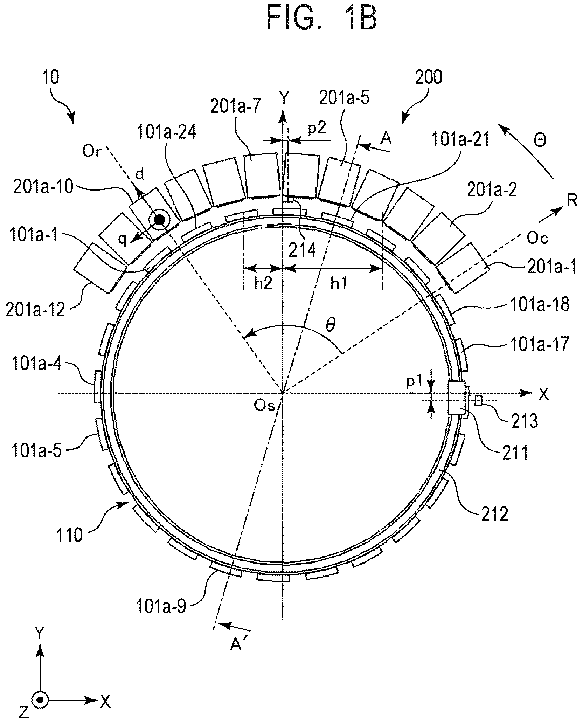

FIG. 1B is a schematic diagram illustrating the rotation drive apparatus according to the first embodiment of the present invention.
[FIG. 1C]

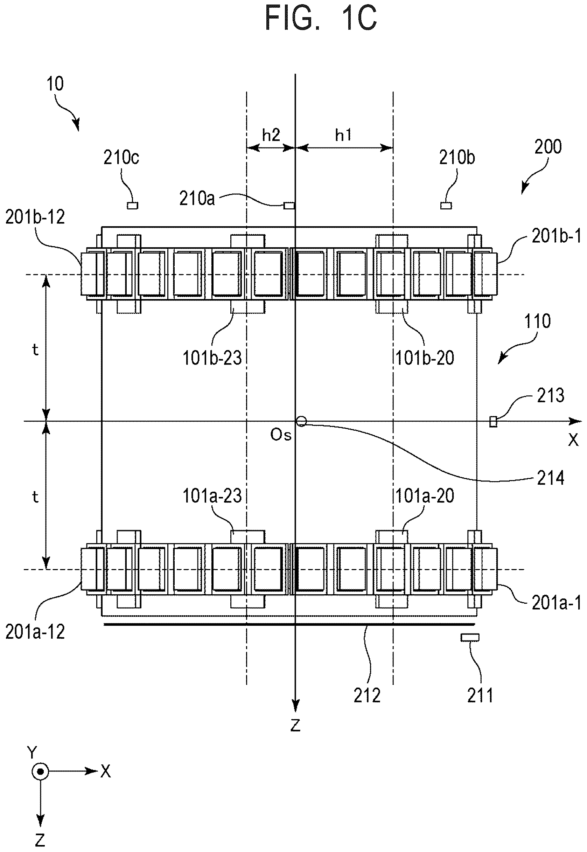

FIG. 1C is a schematic diagram illustrating the rotation drive apparatus according to the first embodiment of the present invention.
[FIG. 1D]

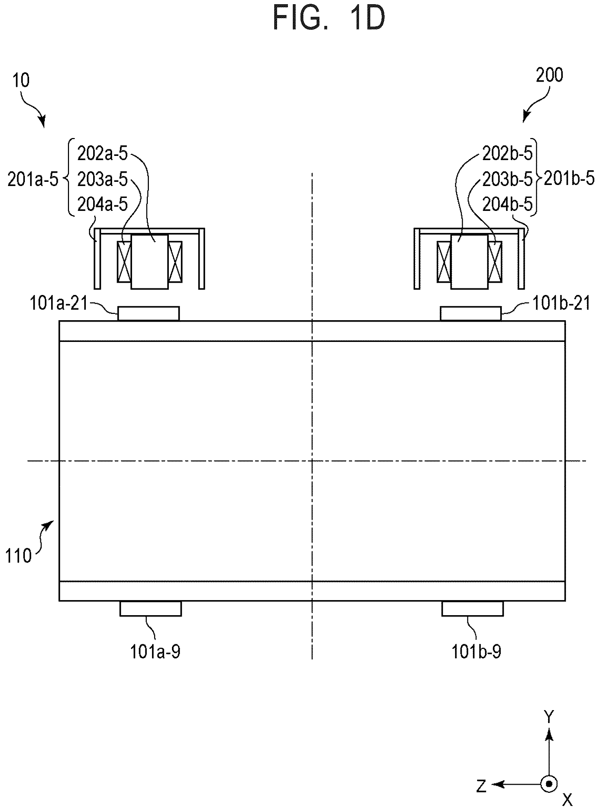

FIG. 1D is a schematic diagram illustrating the rotation drive apparatus according to the first embodiment of the present invention.
[FIG. 2A]

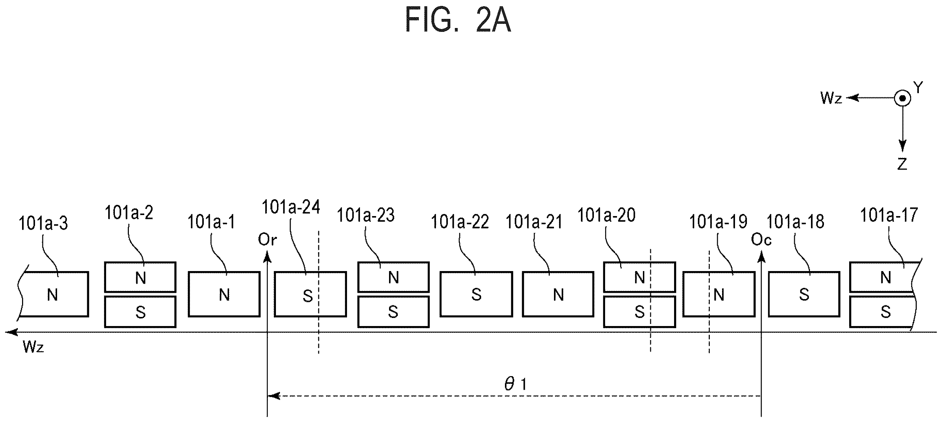

FIG. 2A is a schematic diagram illustrating the arrangement of permanent magnets in the rotation drive apparatus according to the first embodiment of the present invention.
[FIG. 2B]

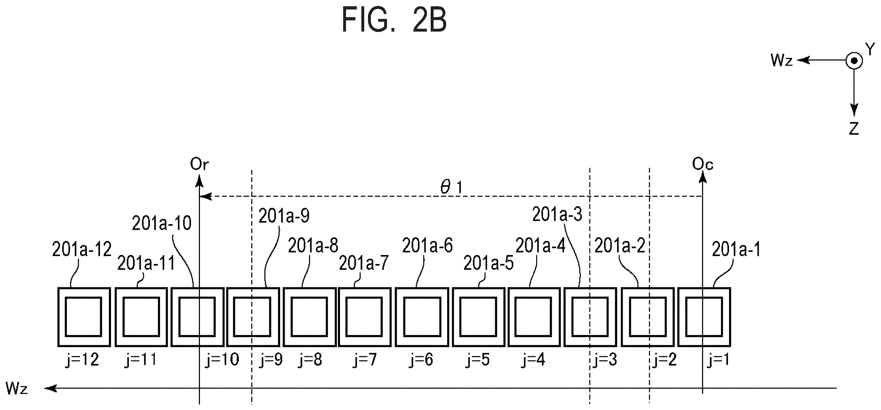

FIG. 2B is a schematic diagram illustrating the arrangement of a coil in a rotation drive apparatus according to a first embodiment of the present invention.
[FIG. 2C]

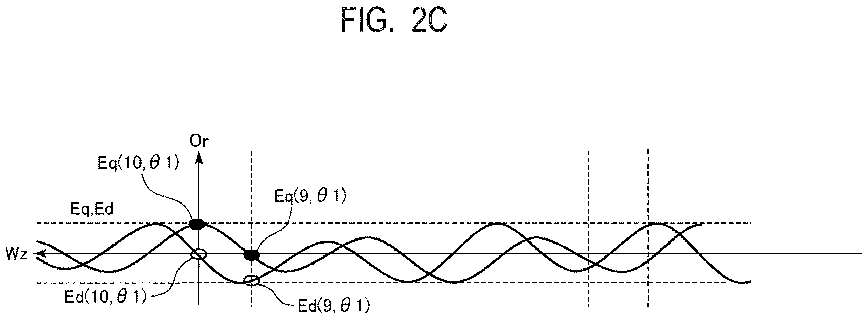

FIG. 2C is a schematic diagram illustrating a thrust constant in the rotation drive apparatus according to the first embodiment of the present invention.
[FIG. 2D]

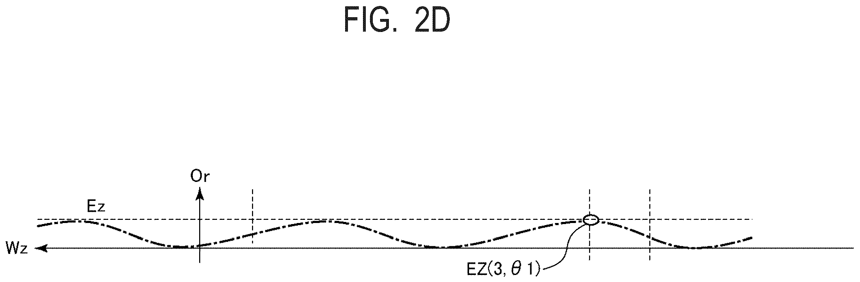

FIG. 2D is a schematic diagram illustrating the thrust constant in the rotation drive apparatus according to the first embodiment of the present invention.
[FIG. 3A]

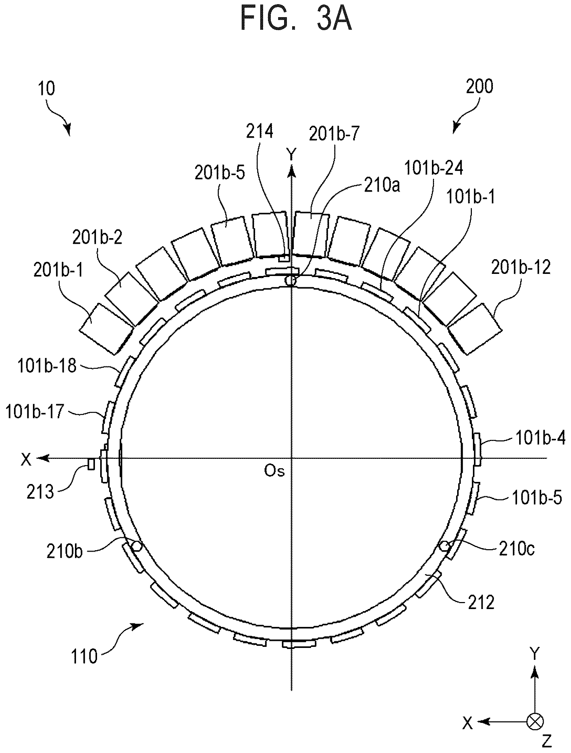

FIG. 3A is a schematic diagram illustrating the arrangement of sensors in the rotation drive apparatus according to the first embodiment of the present invention.

[FIG. 3B]

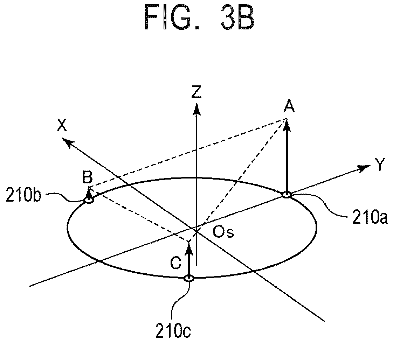

FIG. 3B is a schematic diagram explaining a method of calculating the displacement of a rotor from the sensors in the rotation drive apparatus according to the first embodiment of the present invention.

[FIG. 4]

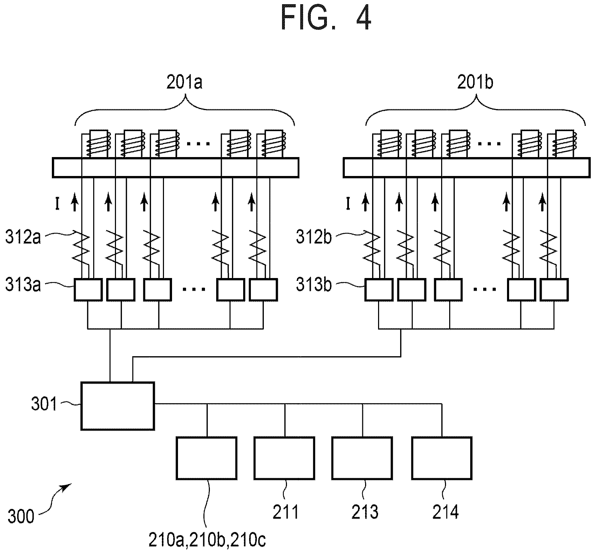

FIG. 4 is a schematic diagram illustrating a current control system in the rotation drive apparatus according to the first embodiment of the present invention.

[FIG. 5]

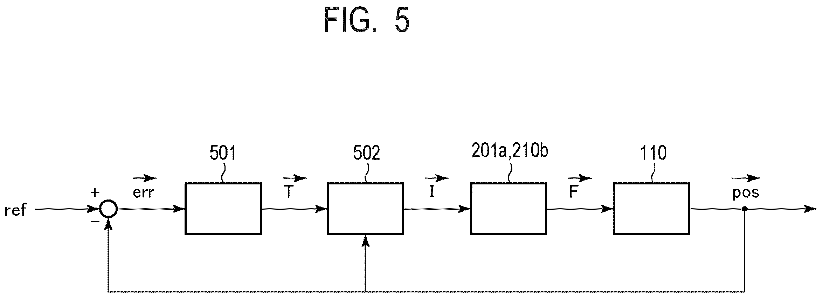

FIG. 5 is a block diagram illustrating a control loop for calculating the magnitude of a torque applied to the rotor in the rotation drive apparatus according to the first embodiment of the present invention.

[FIG. 6]

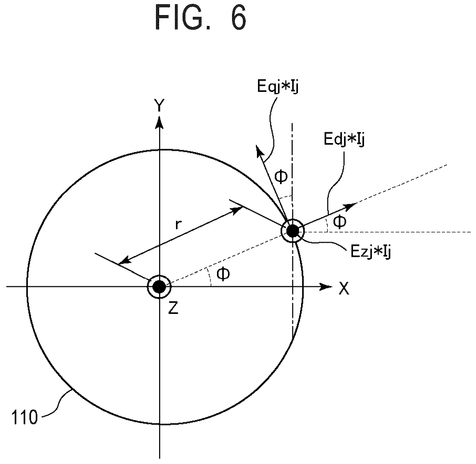

FIG. 6 is a schematic diagram illustrating the torque controlled by the coils in the rotation drive apparatus according to the first embodiment of the present invention.

[FIG. 7A]

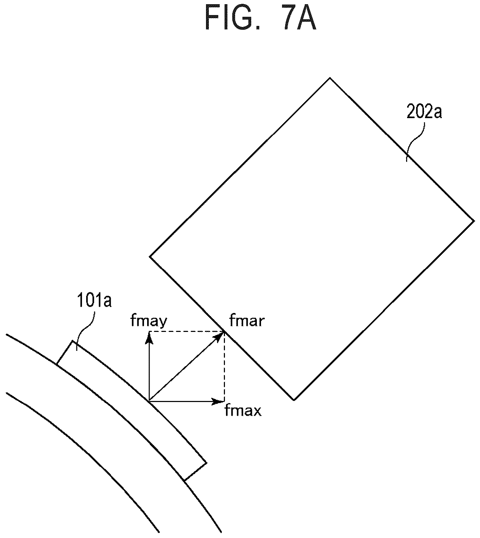

FIG. 7A is a schematic diagram illustrating an attractive force acting between the permanent magnet and the core of the coil in the rotation drive apparatus according to the first embodiment of the present invention.

[FIG. 7B]

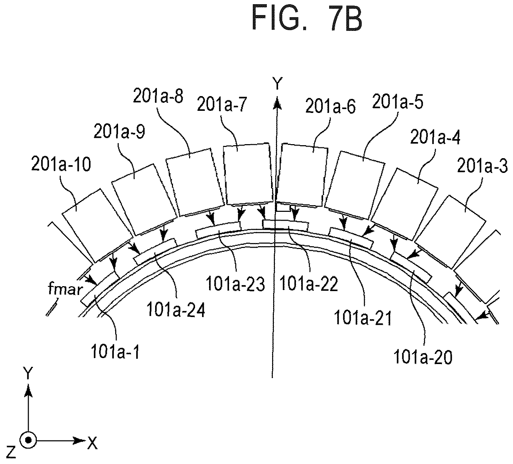

FIG. 7B is a schematic diagram illustrating an attractive force acting between the permanent magnets and the cores of the coils in the rotation drive apparatus according to the first embodiment of the present invention.

[FIG. 8A]

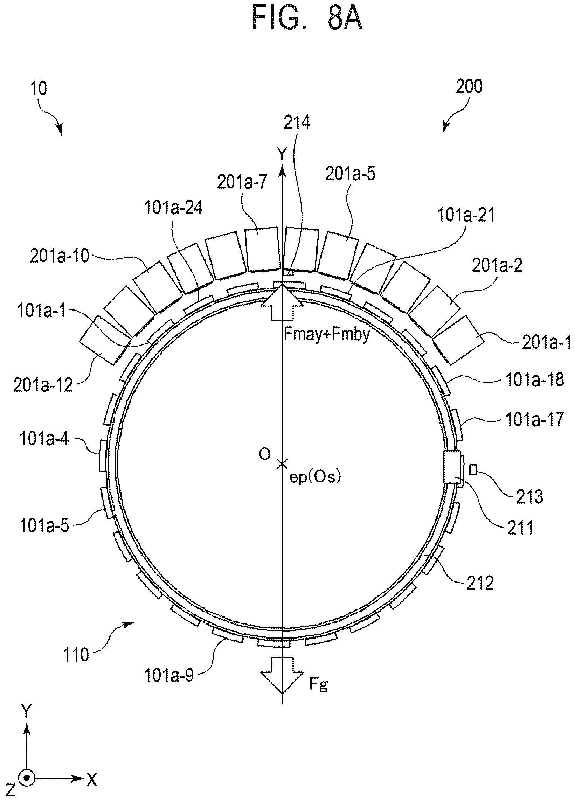

FIG. 8A is a schematic diagram illustrating forces acting according to a position in a Y direction of the rotor in the rotation drive apparatus according to the first embodiment of the present invention.

[FIG. 8B]

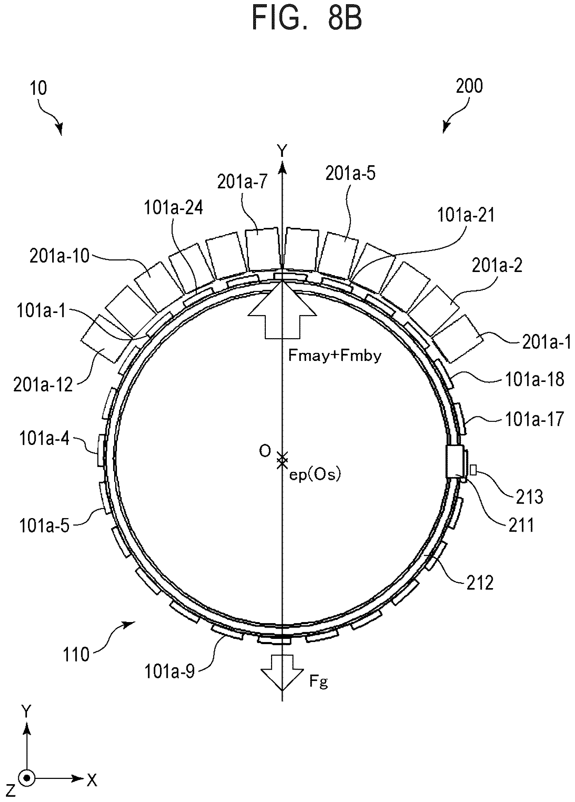

FIG. 8B is a schematic diagram illustrating the forces acting according to a position in the Y direction of the rotor in the rotation drive apparatus according to the first embodiment of the present invention.

[FIG. 8C]

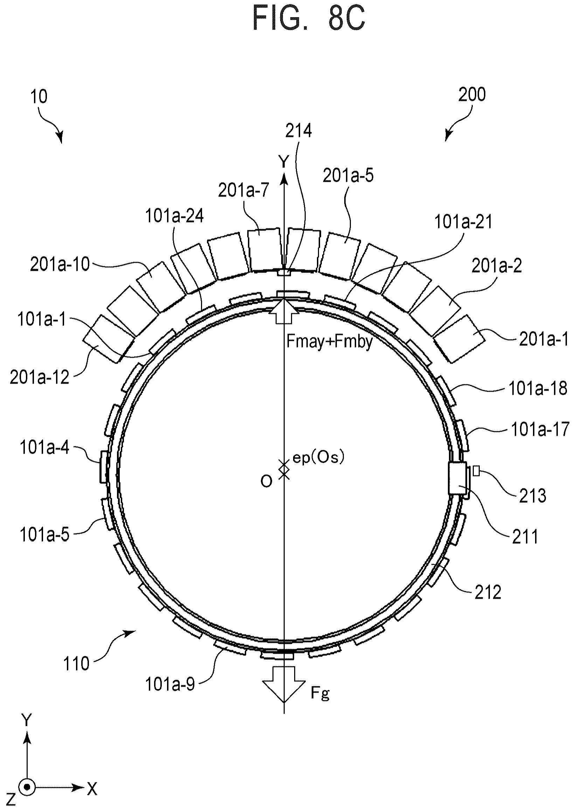

FIG. 8C is a schematic diagram illustrating the forces acting according to a position in the Y direction of the rotor in the rotation drive apparatus according to the first embodiment of the present invention.

[FIG. 9A]

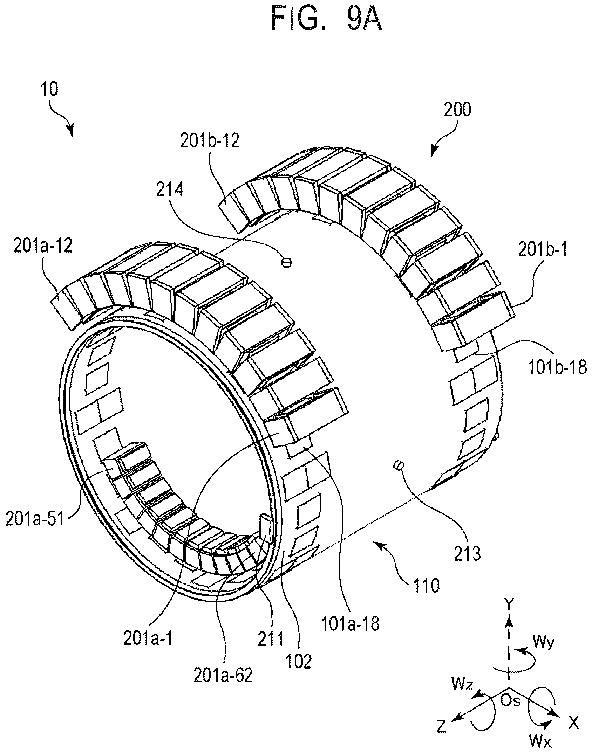

FIG. 9A is a schematic diagram illustrating a rotation drive apparatus according to a second embodiment of the present invention.

[FIG. 9B]

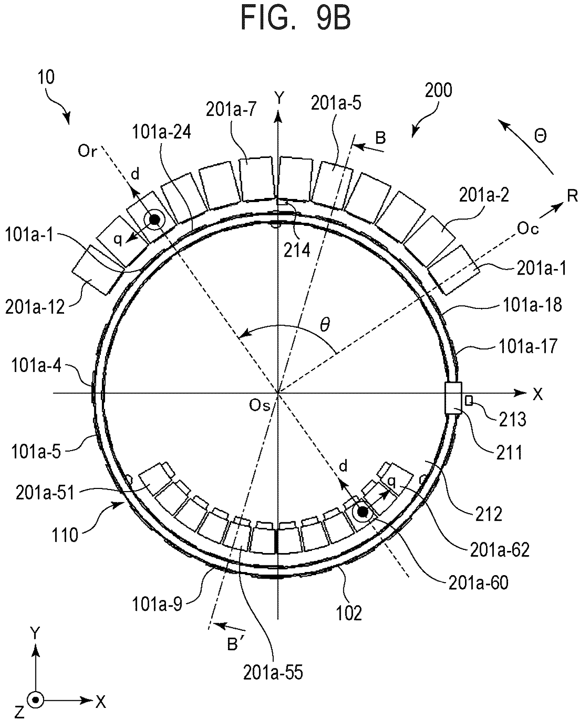

FIG. 9B is a schematic diagram illustrating the rotation drive apparatus according to the second embodiment of the present invention.

[FIG. 9C]

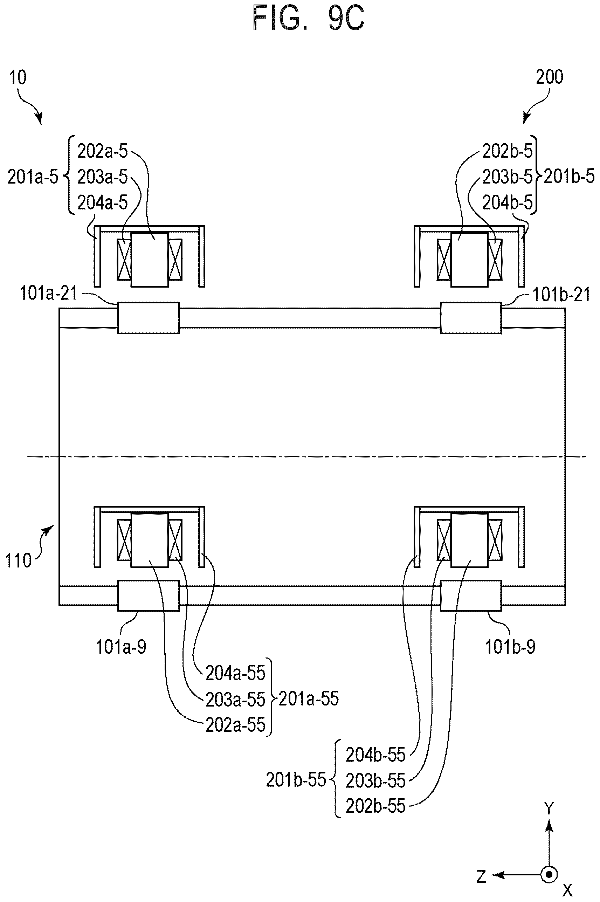

FIG. 9C is a schematic diagram illustrating the rotation drive apparatus according to the second embodiment of the present invention.

[FIG. 10A]

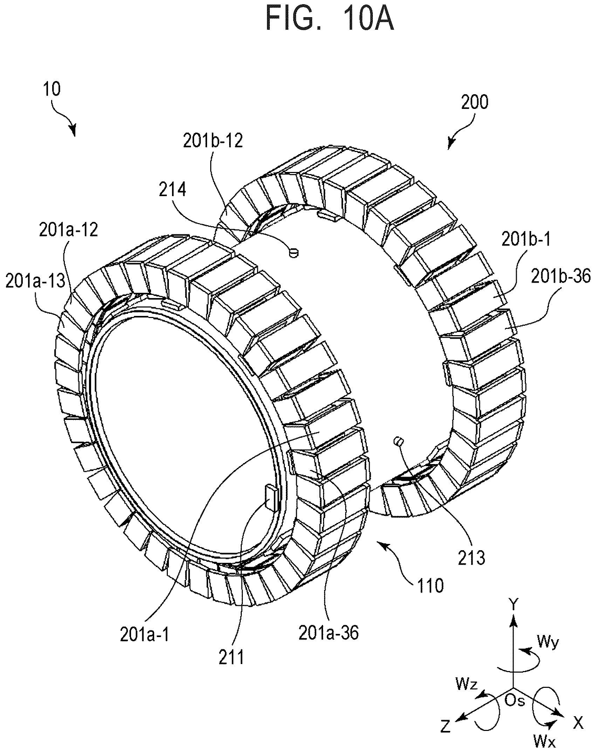

FIG. 10A is a schematic diagram illustrating a rotation drive apparatus according to a third embodiment of the present invention.

[FIG. 10B]

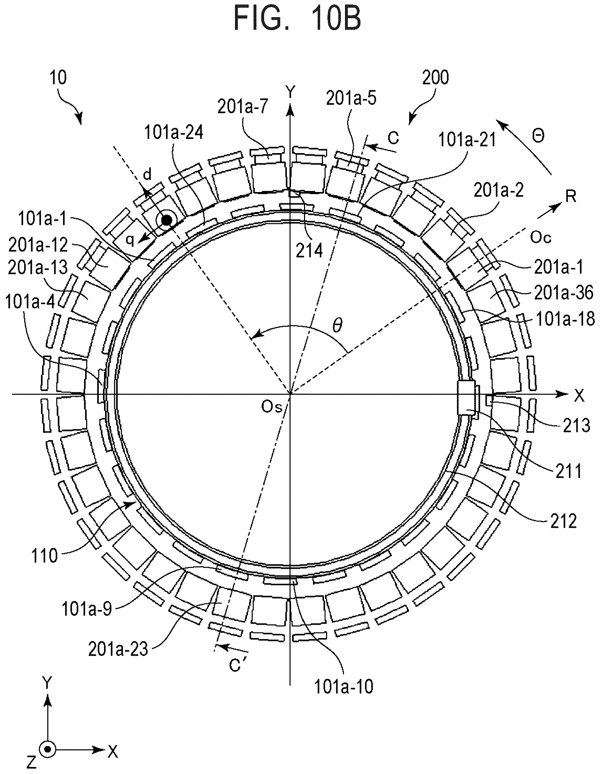

FIG. 10B is a schematic diagram illustrating the rotation drive apparatus according to the third embodiment of the present invention.

[FIG. 10C]

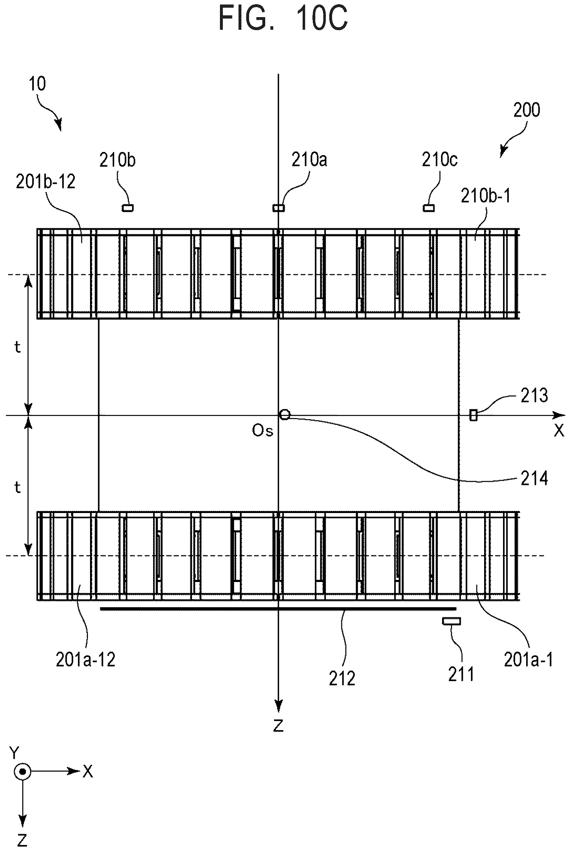

FIG. 10C is a schematic diagram illustrating the rotation drive apparatus according to the third embodiment of the present invention.

[FIG. 10D]

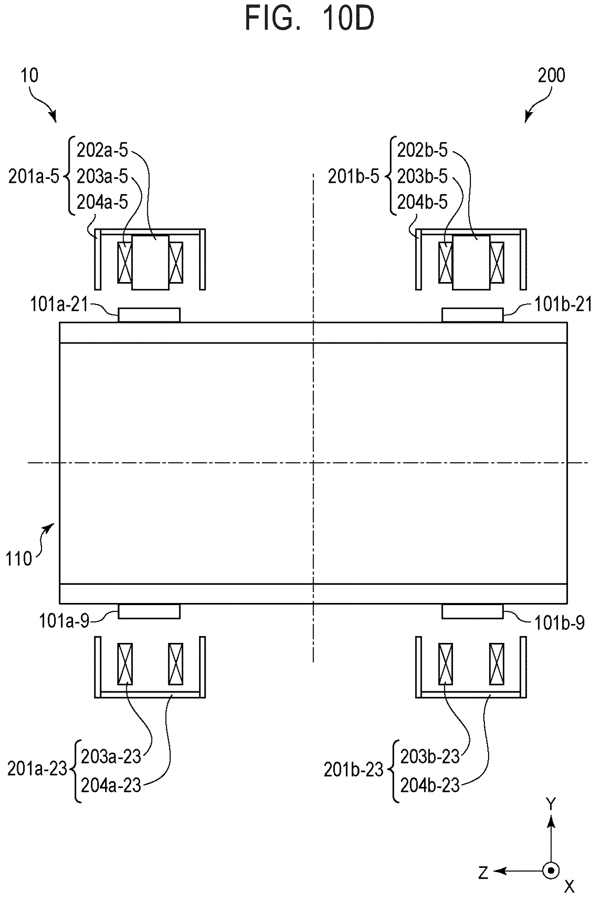

FIG. 10D is a schematic diagram illustrating the rotation drive apparatus according to the third embodiment of the present invention.

[FIG. 11A]

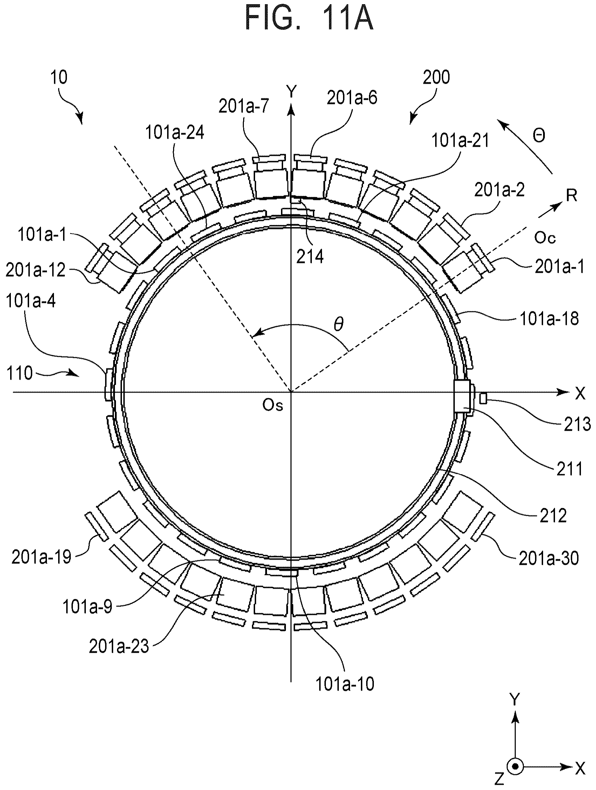

FIG. 11A is a schematic diagram illustrating a rotation drive apparatus according to a modification of the third embodiment of the present invention.

[FIG. 11B]

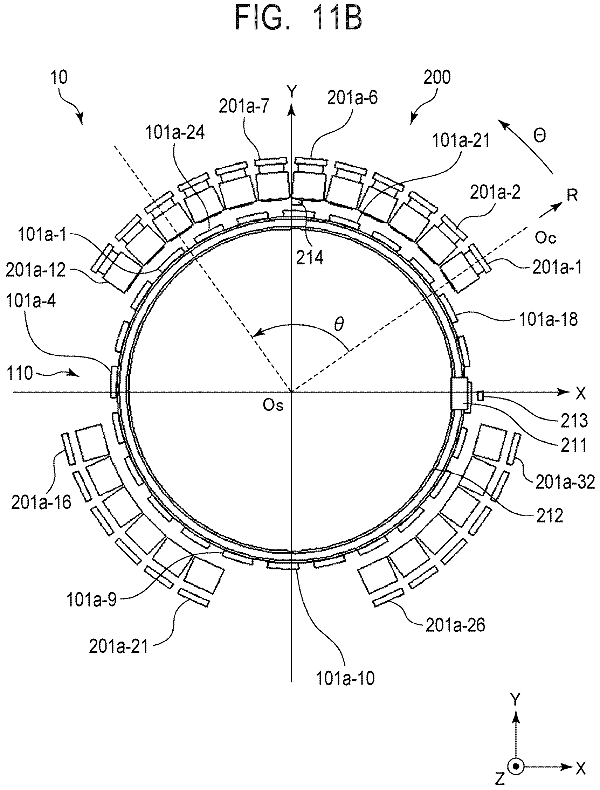

FIG. 11B is a schematic diagram illustrating a rotation drive apparatus according to a modification of the third embodiment of the present invention.

[FIG. 12A]

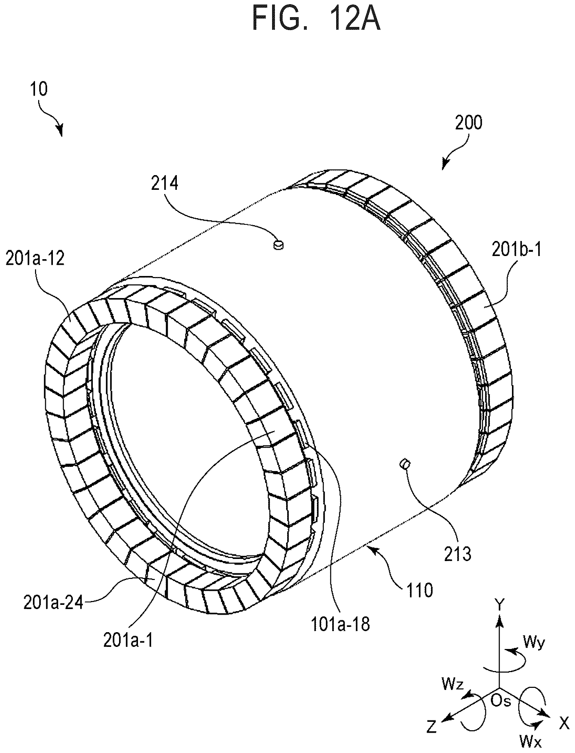

FIG. 12A is a schematic diagram illustrating a rotation drive apparatus according to a fourth embodiment of the present invention.

[FIG. 12B]

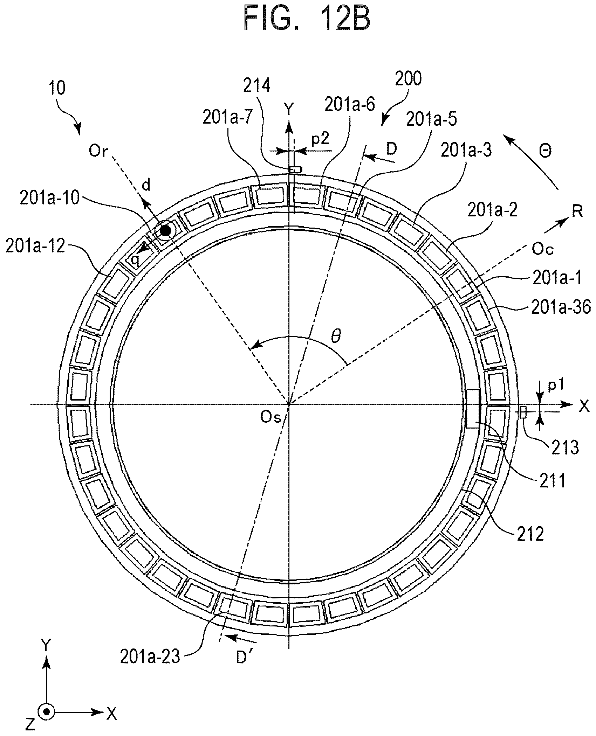

FIG. 12B is a schematic diagram illustrating the rotation drive apparatus according to the fourth embodiment of the present invention.
[FIG. 12C]

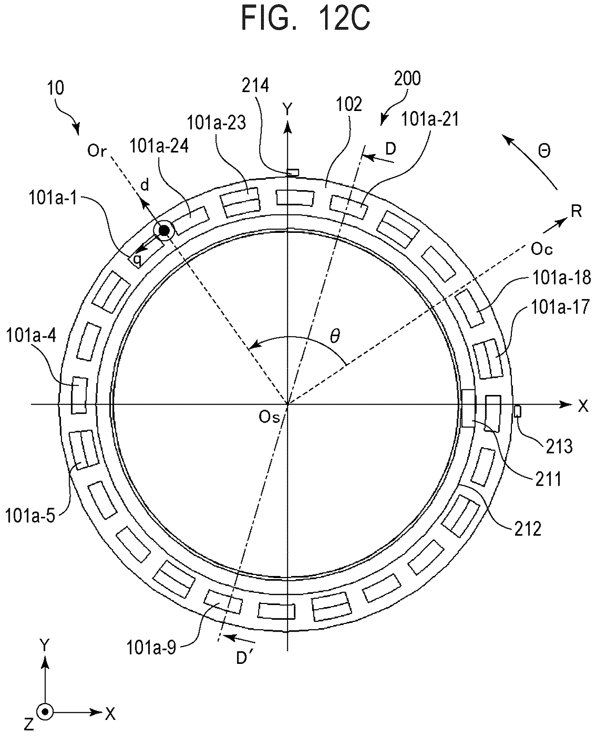

FIG. 12C is a schematic diagram illustrating the rotation drive apparatus according to the fourth embodiment of the present invention.
[FIG. 12D]

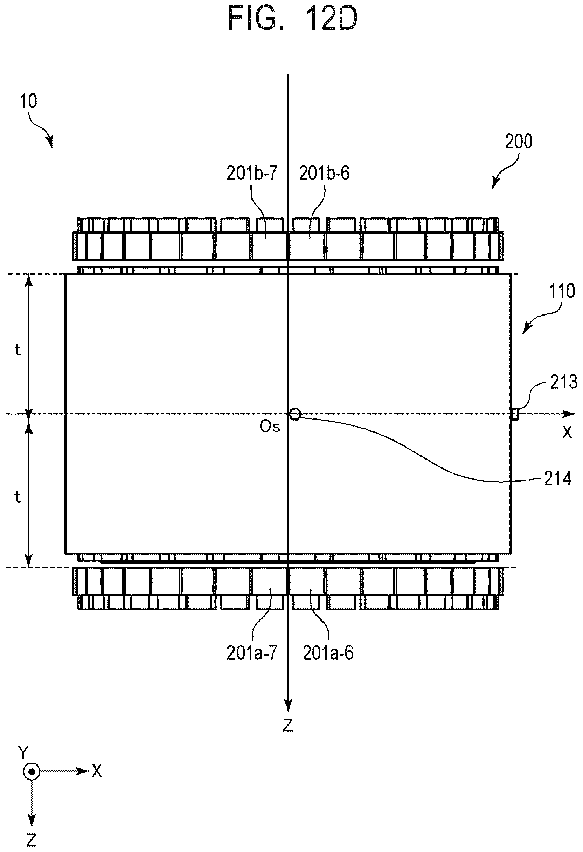

FIG. 12D is a schematic diagram illustrating the rotation drive apparatus according to the fourth embodiment of the present invention.
[FIG. 12E]

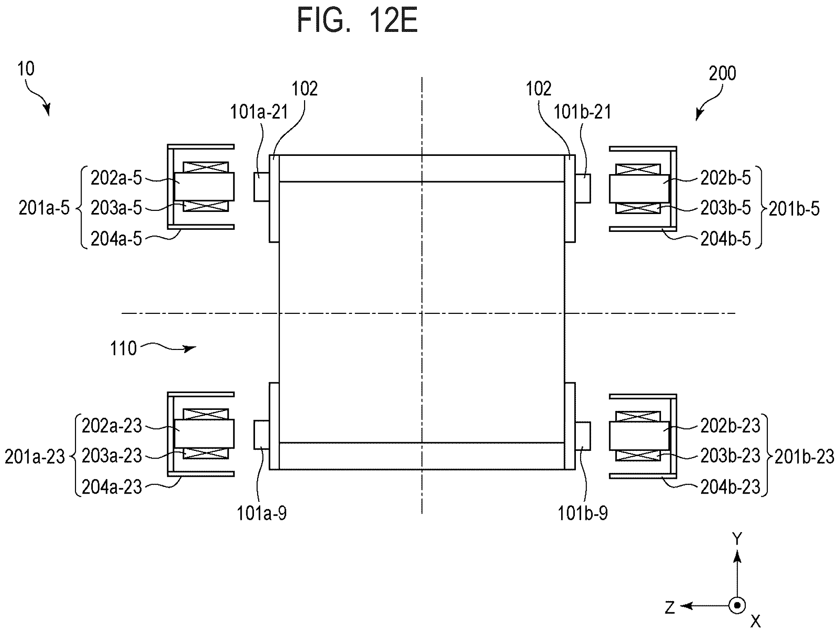

FIG. 12E is a schematic diagram illustrating the rotation drive apparatus according to the fourth embodiment of the present invention.
[FIG. 13A]

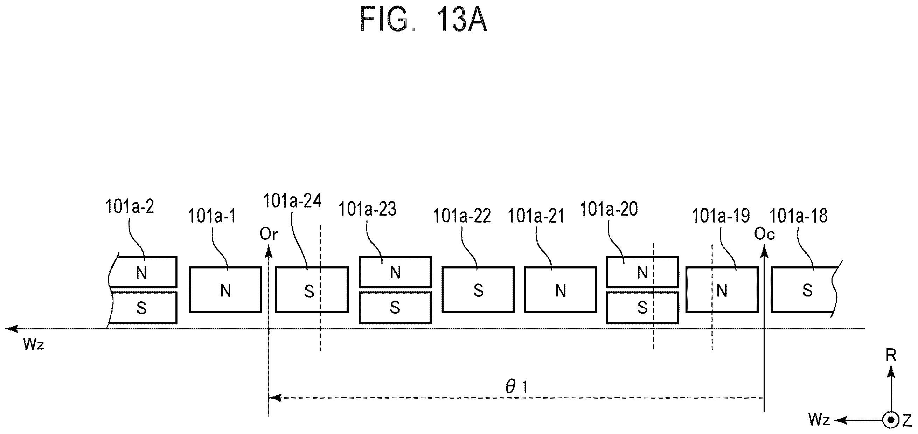

FIG. 13A is a schematic diagram illustrating the arrangement of permanent magnets in the rotation drive apparatus according to the fourth embodiment of the present invention.
[FIG. 13B]

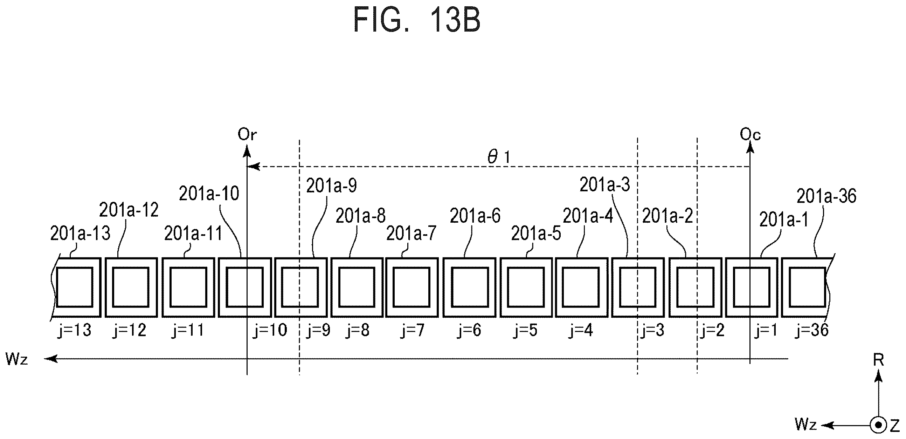

FIG. 13B is a schematic diagram illustrating the arrangement of coils in the rotation drive apparatus according to the fourth embodiment of the present invention.
[FIG. 13C]

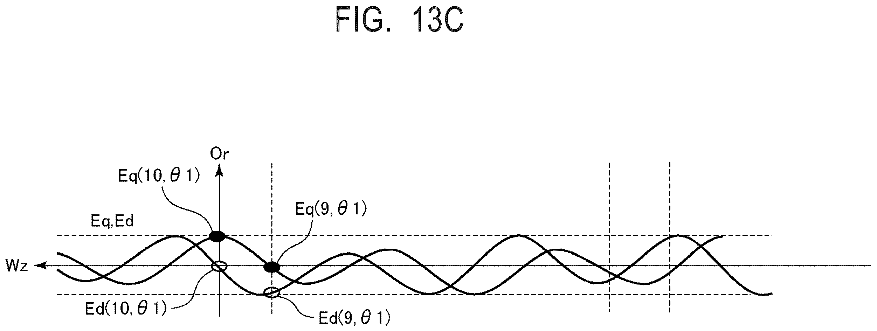

FIG. 13C is a schematic diagram illustrating a thrust constant in the rotation drive apparatus according to the fourth embodiment of the present invention.
[FIG. 13D]

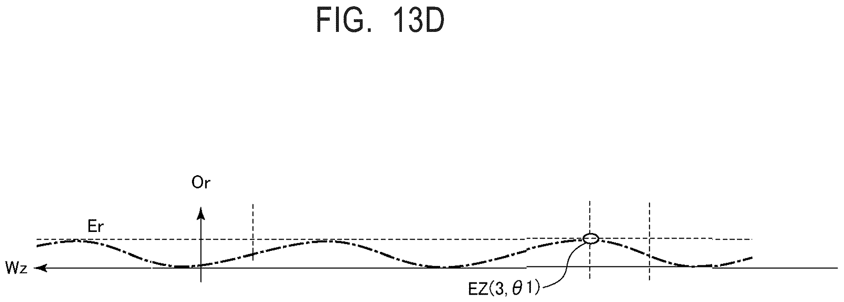

FIG. 13D is a schematic diagram illustrating the thrust constant in the rotation drive apparatus according to the fourth embodiment of the present invention.
[FIG. 14]

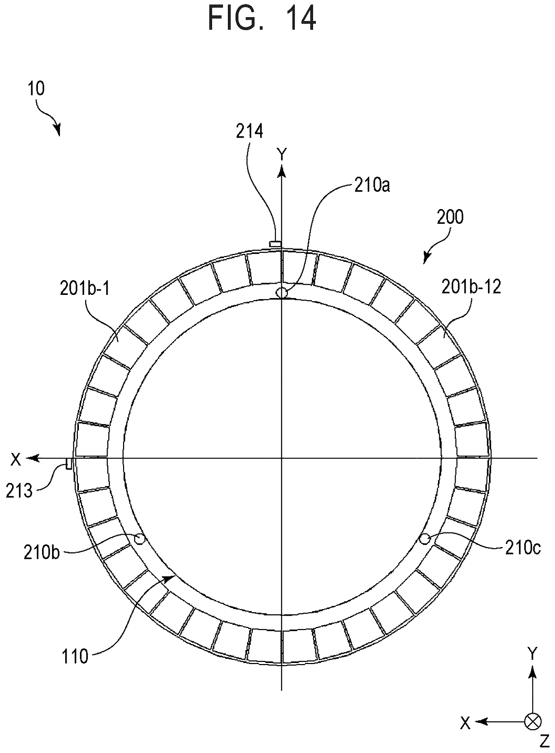

FIG. 14 is a schematic diagram illustrating the arrangement of sensors in the rotation drive apparatus according to the fourth embodiment of the present invention.
[FIG. 15]

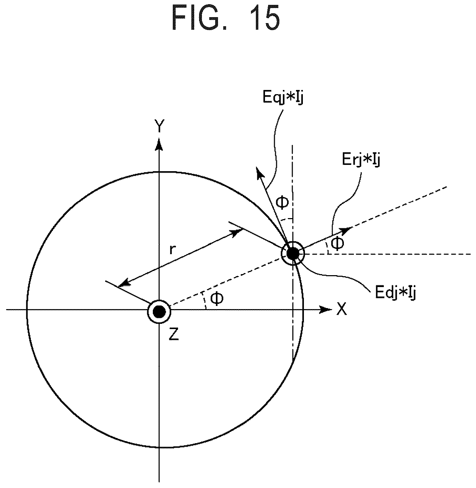

FIG. 15 is a schematic diagram illustrating the torque controlled by the coils in the rotation drive apparatus according to the fourth embodiment of the present invention.
[FIG. 16]

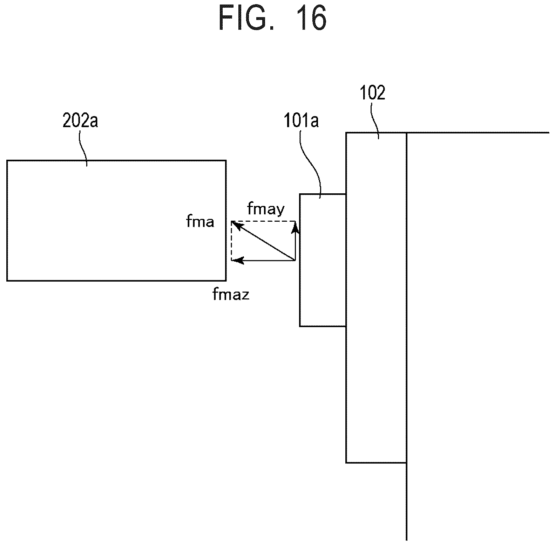

FIG. 16 is a schematic diagram illustrating an attractive force acting between the permanent magnet and the core of the coil in the rotation drive apparatus according to the fourth embodiment of the present invention.
[FIG. 17A]

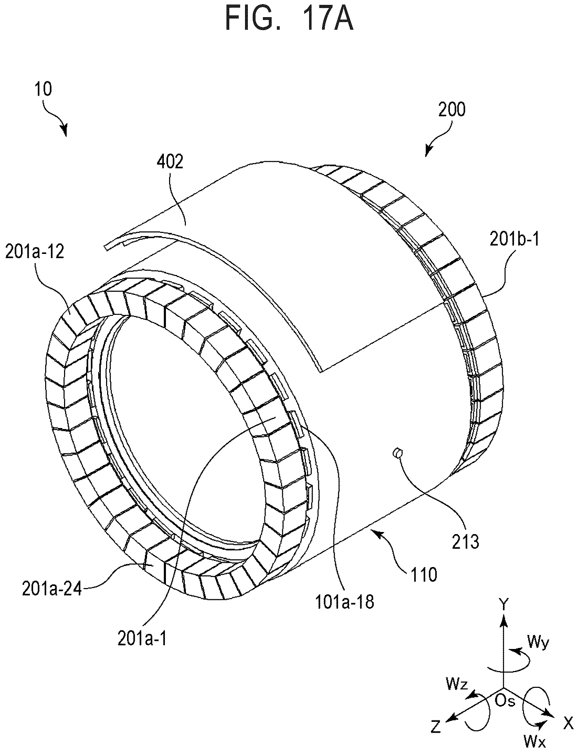

FIG. 17A is a schematic diagram illustrating a rotation drive apparatus according to a fifth embodiment of the present invention.
[FIG. 17B]

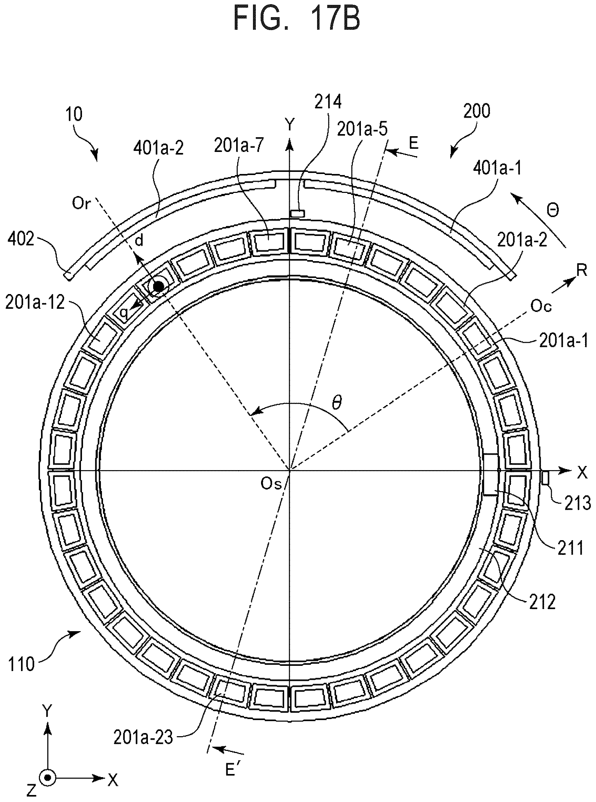

FIG. 17B is a schematic diagram illustrating the rotation drive apparatus according to the fifth embodiment of the present invention.
[FIG. 17C]

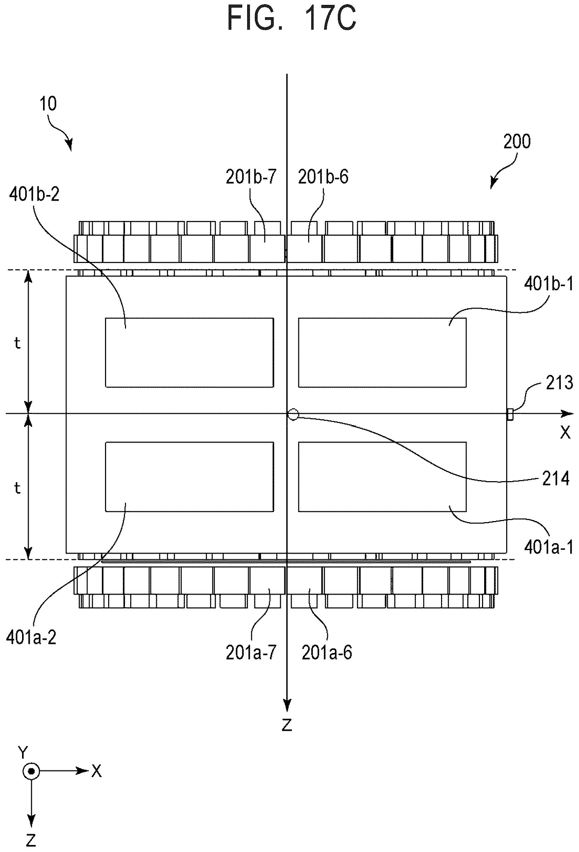

FIG. 17C is a schematic diagram illustrating the rotation drive apparatus according to the fifth embodiment of the present invention.
[FIG. 17D]

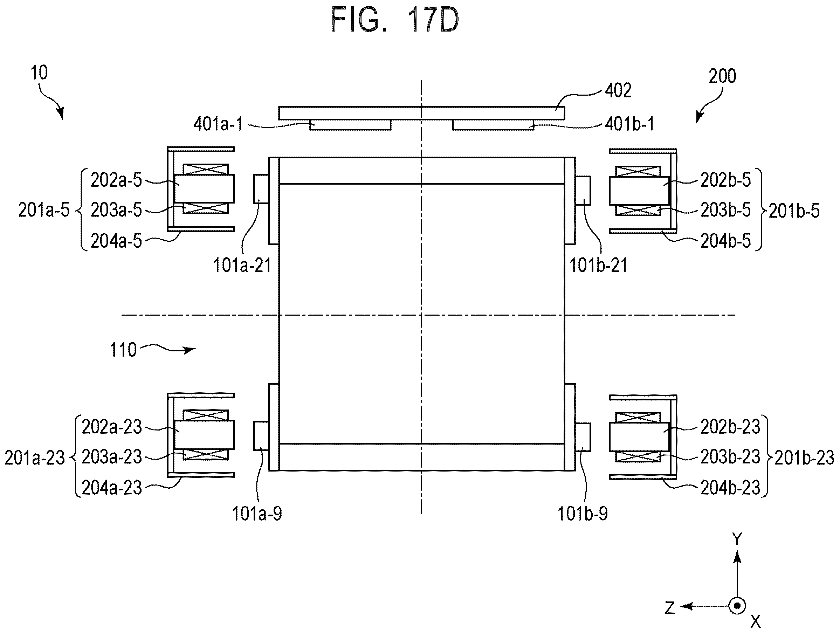

FIG. 17D is a schematic diagram illustrating the rotation drive apparatus according to the fifth embodiment of the present invention.
[FIG. 18A]

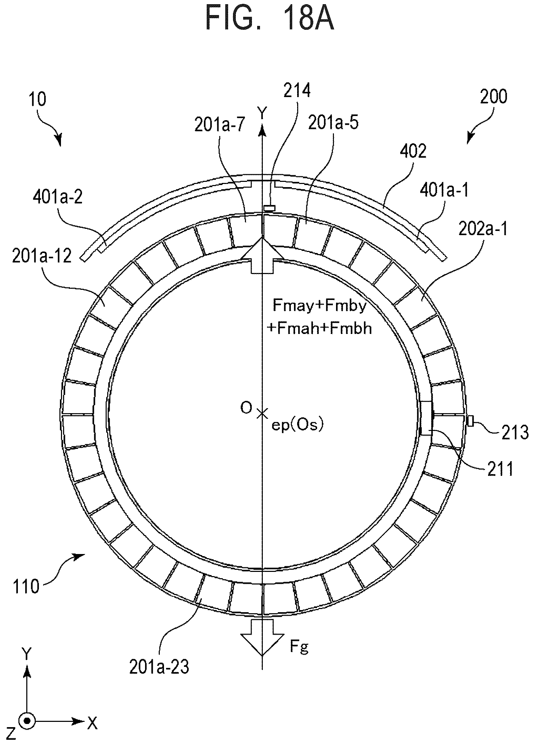

FIG. 18A is a schematic diagram illustrating forces acting according to a position in a Y direction of the rotor in the rotation drive apparatus according to the fifth embodiment of the present invention.
[FIG. 18B]

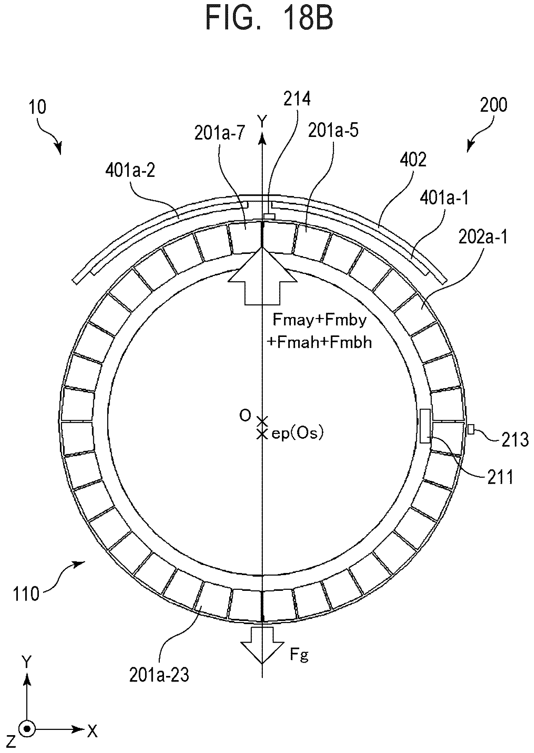

FIG. 18B is a schematic diagram illustrating the forces acting according to a position in the Y direction of the rotor in the rotation drive apparatus according to the fifth embodiment of the present invention.
[FIG. 18C]

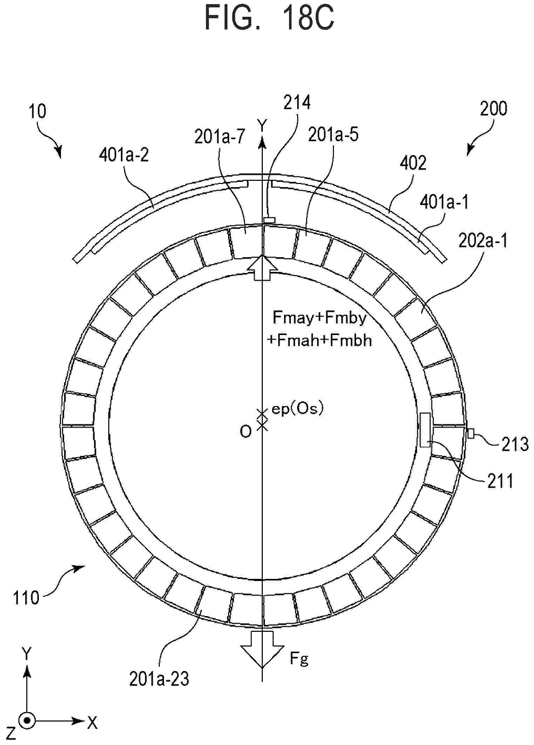

FIG. 18C is a schematic diagram illustrating the forces acting according to a position in the Y direction of the rotor in the rotation drive apparatus according to the fifth embodiment of the present invention.
[FIG. 19A]

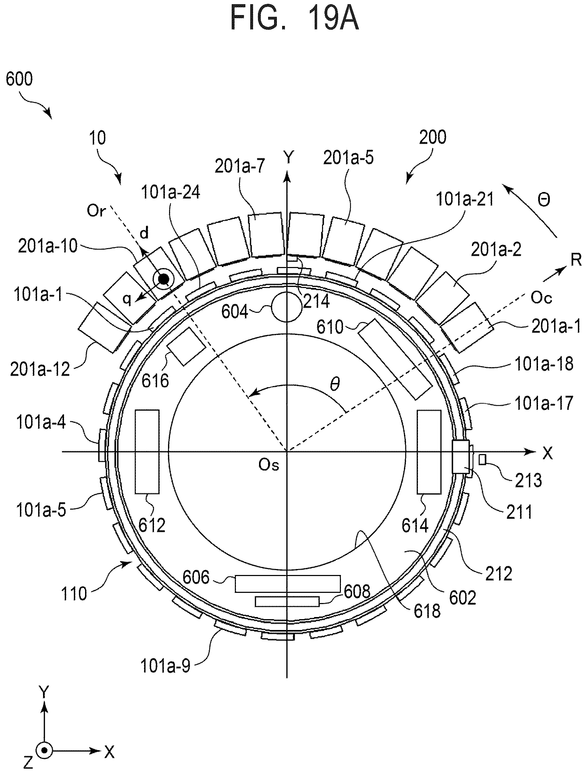

FIG. 19A is a schematic diagram illustrating an example of rotation equipment according to a sixth embodiment of the present invention.
[FIG. 19B]

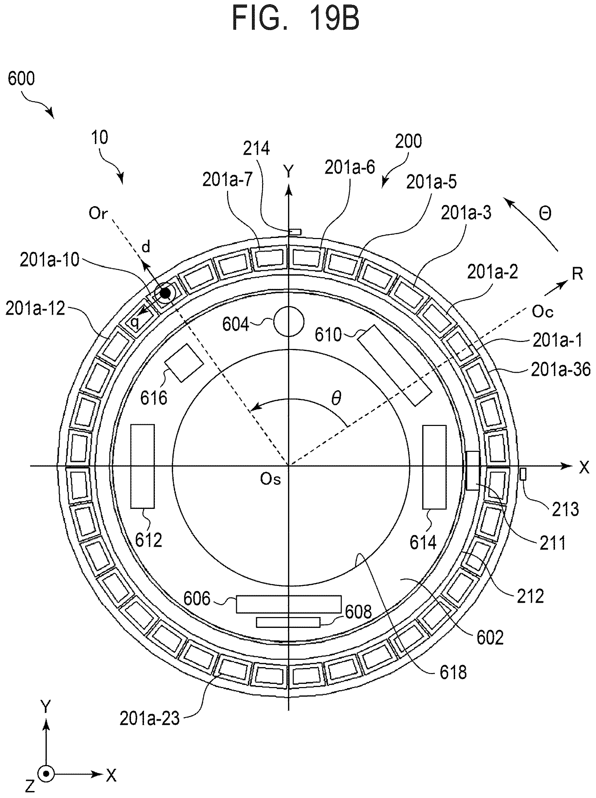

FIG. 19B is a schematic diagram illustrating another example of rotation equipment according to the sixth embodiment of the present invention.

[Description of Embodiments]

[First Embodiment]

A rotation drive apparatus according to a first embodiment of the present invention will be described with reference to FIG. 1A to FIG. 8C. The rotation drive apparatus according to the present embodiment includes a rotor having a rotation axis intersecting the direction of gravity and a stator arranged on the upper side of the rotor in the direction of gravity, and rotates the rotor around the axis in a contactless state while magnetically levitating the rotor in the direction of gravity.

Here, the coordinate axis and direction used in the following description are defined. First, the rotation axis around which the rotor 110 described later rotates is defined as a Z-axis. Further, an XY-plane is taken so as to be orthogonal to the Z-axis, and two axes orthogonal to each other in the XY-plane are defined as an X-axis and a Y-axis, respectively. Further, the direction along the X-axis is defined as an X direction, the direction along the Y-axis is defined as a Y direction, and the direction along the Z-axis is defined as a Z direction. Further, the rotation direction around the X-axis is defined as a Wx direction, the rotation direction around the Y-axis is defined as a Wy direction, and the rotation direction around the Z-axis is defined as a Wz direction. The origin of the XYZ coordinates including the X-axis, the Y-axis, and the Z-axis is set as Os. The origin Os is the position where the center lines of the coils arranged in the stator 200 described later are connected. The positive direction of each rotation in the Wx direction, the Wy direction and the Wz direction is a clockwise direction toward the positive direction of each axis in which each the X-axis, the Y-axis and the Z-axis extends from the origin Os. Further, an R-axis is taken in a radial direction in which a radius increases in a plane along the XY-plane with the Z-axis as the central axis. Further, in the following description, the Z direction is taken as the horizontal direction and the Y direction as the direction of gravity. Note that the X direction, the Y direction and the Z direction are not necessarily limited to directions orthogonal to each other, but can be defined as directions intersecting each other. Also note that the Z direction does not necessarily have to be the horizontal direction, but can be a direction inclined with respect to the horizontal direction. In this case, the X direction and the Z direction can be defined in the same way with respect to the Z direction.

First, the configuration of the rotation drive apparatus according to the present embodiment will be described with reference to FIG. 1A to FIG. 1D. FIG. 1A is a perspective view illustrating the rotation drive apparatus 10 according to the present embodiment. FIG. 1B is a side view of the rotation drive apparatus 10 according to the present embodiment viewed in the Z direction. FIG. 1C is a top view of the rotation drive apparatus 10 according to the present embodiment viewed in the Y direction. FIG. 1D is a cross-sectional view taken along a line A-A' illustrated in FIG. 1B viewed in the X direction.

As illustrated in FIG. 1A to FIG. 1C, the rotation drive apparatus 10 according to the present embodiment includes a rotor 110 and a stator 200. The rotor 110 includes a plurality of first permanent magnets 101a and a plurality of second permanent magnets 101b as permanent magnet groups. The stator 200 includes a plurality of first coils 201a and a plurality of second coils 201b as coil groups. The rotation drive apparatus 10 includes a current control system 300 described later as a control unit that controls the drive of the rotor 110.

Note that, in the following description, each permanent magnet in the plurality of first permanent magnets 101a is simply denoted as "first permanent magnet 101a" and each permanent magnet in the plurality of second permanent magnets 101b is simply denoted as "second permanent magnet 101b", unless otherwise necessary to be specifically distinguished. When it is necessary to identify each permanent magnet individually, Expressions such as "first permanent magnet 101a-1", "first permanent magnet 101a-2", "second permanent magnet 101b-1", "second permanent magnet 101b-2", and the like are used, with numeral signs each further having a branch number following "-" to identify each permanent magnet individually.

Similarly, unless otherwise necessary to be specifically distinguished, each coil in the plurality of first coils 201a is simply denoted as "first coil 201a" and each coil in the plurality of second coils 201b is simply denoted as "second coil 201b". When it is necessary to identify each coil individually, Expressions such as "first coil 201a-1", "first coil 201a-2", "second coil 201b-1", "second coil 201b-2", and the like, are used, with numeral signs each further having a branch number following "-" to identify each coil individually. The components of the coil are also denoted each with a numeral sign having a branch number in the same manner to be identified individually as necessary. For the coil group of the plurality of first coils 201a or the plurality of second coils 201b, a symbol "j" as an index of the coil is used as described later. Note that j is a positive integer.

As illustrated in FIG. 1A, the rotor 110 has a hollow cylindrical shape with the Z axis as its central axis. The rotor 110 is configured to be rotatable in the Wz direction, which is a rotation direction with the Z-axis as a rotation axis. Note that the shape of the rotor 110 is not limited to the hollow cylindrical shape. The shape of the rotor 110 may be a shape that can be rotatable around a rotation axis along a direction intersecting the direction of gravity, and may be another shape such as a cylindrical shape depending on the configuration of equipment that utilizes the rotation of the rotor 110, and the like.

The plurality of first permanent magnets 101a, which are a group of permanent magnets, are mounted and installed on the outer peripheral side surface of the rotor 110 so as to be uniformly arranged in one line in a circumferential shape along the Wz direction, which is the rotation direction. The plurality of second permanent magnets 101b, which are a group of permanent magnets, are also mounted and installed on the outer peripheral side surface of the rotor 110 so as to be uniformly arranged in one line in a circumferential shape along the Wz direction. For example, the plurality of first permanent magnets 101a are circumferentially arranged on the outer peripheral side of one end in the Z direction of the rotor 110, and the plurality of second permanent magnets 101b are circumferentially arranged on the outer peripheral side of the other end in the Z direction of the rotor 110. Thus, the plurality of first permanent magnets 101a and the plurality of second permanent magnets 101b are arranged in the rotor 110.

The plurality of first coils 201a and the plurality of second coils 201b are arranged on the stator 200 so that the plurality of first coils 201a and the plurality of second coils 201b are positioned to face the first permanent magnets 101a and the second permanent magnets 101b above the rotor 110 in the Y direction outside the rotor 110. The plurality of first coils 201a are mounted in positions where they can face the plurality of first permanent magnets 101a of the rotor 110 so as to be uniformly arranged in one line in an arc shape along the Wz direction. The plurality of second coils 201b are mounted in positions where they can face the plurality of second permanent magnets 101b of the rotor 110 so as to be uniformly arranged in one line in an arc shape along the Wz direction. Thus, the plurality of first coils 201a and the plurality of second coils 201b are arranged in the stator 200.

The set of the first coils 201a and the first permanent magnets 101a, and the set of the second coils 201b and the second permanent magnets 101b are arranged on the stator 200 and the rotor 110 symmetrically with the XY-plane as the plane of symmetry. Thus, the rotor 110 is arranged on the lower side of the plurality of first coils 201a and the plurality of second coils 201b in the direction of gravity, and rotates around the Z-axis by rotation control while levitating in the Y direction by levitation control.

In the present embodiment, the first coils 201a-1 to 201a-12 are arranged in order in a Θ direction, which is the circumferential direction along the Wz direction, with a reference Oc, which is a straight line extending obliquely upward along the XY-plane from the origin Os, as the reference of the stator 200. Similarly, the second coils 201b-1 to 201b-12 are arranged in order in the Θ direction with the reference Oc as the reference of the stator 200. Further, the first permanent magnets 101a-1 to 101a-24 are arranged in order in the Θ direction with a reference Or, which is a straight line extending from the origin Os toward a predetermined position on the outer peripheral side surface of the rotor 110, as the reference of the rotor 110. Similarly, the second permanent magnets 101b-1 to 101b-24 are arranged in order in the Θ direction with the reference Or as the reference of the rotor 110.

Note that the number of the first coils 201a, the number of the second coils 201b, the number of the first permanent magnets 101a, and the number of the second permanent magnets 101b are not limited to those illustrated in the present embodiment. The number of the first coils 201a, the number of the second coils 201b, the number of the first permanent magnets 101a, and the number of the second permanent magnets 101b can be suitably changed according to thrusts required in a q-axis direction, a d-axis direction, and the Z direction, and the like, which will be described later. Also note that, although the number of the first coils 201a and the number of the second coils 201b are equal to each other, they do not necessarily have to be equal to each other. Also note that, although the number of the first permanent magnets 101a and the number of the second permanent magnets 101b are equal to each other, they do not necessarily have to be equal to each other.

In the present embodiment, a case will be described in which lines of sets of the permanent magnets and the coils facing each other are two lines including the line of the set of the first permanent magnets 101a and the first coils 201a and the line of the set of the second permanent magnets 101b and the second coils 201b. However, the lines of the set of the permanent magnets and the coils are not limited to this case. In accordance with the rotational accuracy required for the rotor 110, the line of the set of the permanent magnets and the coils may be one line, or may be not only two lines but also three or more lines in which lines of the same set as the set of the first permanent magnets 101a and the first coils 201a are further provided. Note that, when the line of the set of the permanent magnets and the coils is one line, it is desirable that the coils are arranged in plane symmetry with the YZ-plane as the plane of symmetry from the viewpoint of stably controlling the rotor 110.

FIG. 1B is a side view of FIG. 1A viewed from the Z direction. Hereinafter, the first coils 201a and the first permanent magnets 101a will be described with reference to FIG. 1B. Note that the second coil 201b has the same configuration as the first coil 201a, and the second permanent magnet 101b has the same configuration as the first permanent magnet 101a.

As illustrated in FIG. 1B, the plurality of first permanent magnets 101a are mounted and installed along the circumferential direction of the rotor 110 so as to line up on the outer peripheral side surface of the rotor 110. The rotor 110 has a yoke 102 installed on its outer peripheral side surface. The plurality of first permanent magnets 101a are attached to the outer peripheral side surface of the rotor 110 via the yoke 102. Thus, the yoke 102 is arranged on the rear side of the first permanent magnets 101a. The yoke 102 is made of a metal having magnetism, such as iron, SUS400 series stainless steel, or the like to increase the magnetic force of the first permanent magnet 101a. The yoke 102 itself has strength and may also serve as a base member of the rotor 110. The yoke 102 may be installed separately from or integrated with the base member of the rotor 110. Note that it is not necessary for the yoke 102 to have the rotor 110 installed and the yoke 102 may not be installed. Even in this case, it is possible to perform levitation control and rotation control of the rotor 110.

Here, the rotation angle, which is an angle in the Wz direction of the rotor 110, is set to $\theta$. The rotation angle $\theta$ is an angle from the reference Oc in the Wz direction on the side of the stator 200 to the reference Or in the Wz direction on the side of the rotor 110. Note that the reference Oc in the Wz direction on the side of the stator 200 is a straight line connecting the first coil 201a-1 arranged in an arc shape and the origin Os. The reference Or in the Wz direction on the side of the rotor 110 is a straight line connecting the middle of the first permanent magnet 101a-1 and the first permanent magnet 101a-24 and the origin Os.

FIG. 1C is a top view of FIG. 1A viewed from the Y direction. Note that, in FIG. 1C, side yokes 204a and 204b described later are omitted to illustrate the arrangement of the first coils 201a and the second coils 201b for ease of explanation. As illustrated in FIG. 1C, the plurality of first coils 201a and the plurality of second coils 201b are mounted and installed in plane symmetry with the YZ-plane as the plane of symmetry in the stator 200. Further, the plurality of first coils 201a and the plurality of second coils 201b are mounted and arranged in plane symmetry with the XY-plane as the plane of symmetry in the stator 200.

Further, the stator 200 includes an X sensor 213, a Y sensor 214, Z sensors 210a, 210b, and 210c, and a Wz sensor 211 as sensors for detecting the displacement or the attitude of the rotor 110. The displacement of the rotor 110 in the X direction can be detected based on the detection value of the X sensor 213. The displacement of the rotor 110 in the Y direction can be detected based on the detection value of the Y sensor 214. The displacement in the Z direction, the displacement in the Wx direction, and the displacement in the Wy direction of the rotor 110 can be detected based on the detected values of the Z sensors 210a, 210b, and 210c. The displacement of the rotor 110 in the Wz direction can be detected based on the detected values of the Wz sensor 211. The detection of the angular displacement is performed by a motor controller 301 described later.

The X sensor 213 detects the distance in the X direction between the rotor 110 and the X sensor 213. The Y sensor 214 detects the distance in the Y direction between the rotor 110 and the Y sensor 214. The Z sensor 210a detects the distance in the Z direction between the rotor 110 and the Z sensor 210a. The Z sensor 210b detects the distance in the Z direction between the rotor 110 and the Z sensor 210b. The Z sensor 210c detects the distance in the Z direction between the rotor 110 and the Z sensor 210c. The X sensor 213, Y sensor 214, and Z sensors 210a, 210b, and 210c are not limited to any particular sensor, but may be eddy current sensors, displacement sensors, or other sensors that can detect distance. Further, optical sensors, magnetic sensors, or the like may be selected as these sensors with a separate scale for detection.

The X sensor 213 is positioned laterally in the X direction with respect to the rotor 110 so as to face the outer peripheral side surface of the rotor 110. The Y sensor 214 is positioned upward in the Y direction with respect to the rotor 110 so as to face the outer peripheral side surface of the rotor 110. The Z sensors 210a, 210b, 210c are positioned laterally in the Z direction with respect to the rotor 110 so as to face the circumferential end surface of the rotor 110 in the Z direction.

The X sensor 213 and the Y sensor 214 are installed so as to perform detection at a position deviated from the vertex of the outer peripheral side surface of the rotor 110 in the Y direction or the X direction in order to perform detection of the outer peripheral side surface which is the curved surface portion of the rotor 110, respectively. This is due to the following reasons for the X sensor 213 and the Y sensor 214.

The reason for the X sensor 213 is as follows. When the rotor 110 is levitated and controlled, if the X sensor 213 is installed so as to detect the vertex of the outer peripheral side surface in the X direction, the X sensor 213 has a position where the X sensor 213 exhibits the same detection values on the positive side and the negative side in the Y direction when the rotor 110 is controlled to move in the Y direction. In order to avoid such the same detection values, as illustrated in FIG. 1B, the X sensor 213 is installed at a position offset by an offset amount p1 on the negative side in the Y direction from the position facing the vertex of the outer peripheral side surface of the rotor 110 in the X direction.

Further, the X sensor 213 is arranged so that the range of the levitation control in the Y direction of the rotor 110 is equal to or less than the offset amount p1. Since the X sensor 213 is arranged in this manner, the X sensor 213 does not exceed the vertex of the outer peripheral side surface of the rotor 110 in the X direction in terms of configuration or control in the Y direction, the X sensor 213 does not exhibit the same detection values on the positive side and the negative side in the Y direction.

Since the position of the X sensor 213 is offset in the Y direction from the position where the X sensor 213 faces the vertex of the outer peripheral side face of the rotor 110 in the X direction, the position of the rotor 110 can be accurately detected by the X sensor 213. Note that, although the X sensor 213 is offset in the negative side in the Y direction in FIG. 1B, the X sensor 213 may be offset in the positive side in the Y direction.

The reason for the Y sensor 214 is as follows. Similarly, when the rotor 110 is levitated and controlled, if the Y sensor 214 is installed so as to detect the vertex of the outer peripheral side surface in the Y direction, the Y sensor 214 has a position where the Y sensor 214 exhibits the same detection values on the positive side and the negative side in the X direction when the rotor 110 is controlled to move in the X direction. In order to avoid such the same detection values, as illustrated in FIG. 1B, the Y sensor 214 is installed at a position offset by an offset amount p2 on the positive side in the X direction from the position facing the vertex of the outer peripheral side surface of the rotor 110 in the Y direction.

Further, the Y sensor 214 is arranged so that the range of the movement control in the X direction of the rotor 110 is equal to or less than the offset amount p2. Since the Y sensor 214 is arranged in this manner, the Y sensor 214 does not exceed the vertex of the outer peripheral side surface of the rotor 110 in the Y direction in terms of configuration or control in the X direction, the Y sensor 214 does not exhibit the same detection value on the positive side and the negative side in the X direction.

Since the position of the Y sensor 214 is offset in the X direction from the position where the Y sensor 214 faces the vertex of the outer peripheral side face of the rotor 110 in the Y direction, the position of the rotor 110 can be accurately detected by the Y sensor 214. Note that, although the Y sensor 214 is offset in the positive side in the X direction in FIG. 1B, the Y sensor 214 may be offset in the negative side in the X direction.

However, when the position of the rotor 110 is simultaneously measured by the X sensor 213 and the Y sensor 214, the displacement of the rotor 110 can be detected. Therefore, it is not necessary that the X sensor 213 and the Y sensor 214 are offset as described above.

The Wz sensor 211 is positioned on the side opposite to the Z sensors 210a, 210b, and 210c in the Z direction with respect to the rotor 110 so as to face the circumferential end surface of the rotor 110 in the Z direction. A scale 212 is mounted and installed on the circumferential end surface of the rotor 110 which the Wz sensor 211 faces. The Wz sensor 211 detects the rotation angle of the rotor 110 in the Wz direction by reading a pattern on the scale 212. The Wz sensor 211 may be any sensor capable of detecting the angle of an optical type, a magnetic type, or the like, and may be of an absolute type or an increment type used with origin detection means.

FIG. 1D is a cross-sectional view illustrating the cross section along the line A-A' illustrated in FIG. 1B. Here, the structure and mounting position of the first coil 201a and the second coil 201b will now be described with reference to FIG. 1D. The first coil 201a and the second coil 201b are each formed by winding a conductor wire around a magnetic material such as a silicon steel sheet or the like used in general motors.

Specifically, as illustrated in FIG. 1D, the first coil 201a-5 is formed by winding a winding 203a-5 around a core 202a-5 formed of a magnetic material such as a silicon steel sheet and attaching a U-shaped side yoke 204a-5 to the core 202a-5. The side yoke 204a-5, like the core 202a-5, is formed of a magnetic material such as a silicon steel sheet. The core 202a-5 is arranged so that its central axis faces the Z-axis along the XY-plane. The winding 203a-5 is wound around the core 202a-5 with the central axis of the core 202a-5 as the center. The side yoke 204-5 is composed of three plate-like parts covering the outer side in the R-axis direction and both sides in the Z direction of the core 202a-5. All of the other first coils 201a have the same configuration as the first coils 201a-5.

Note that, although the configuration with the side yoke 204a attached is described as an example in the present embodiment, it is not limited to this configuration. The side yoke 204a only needs to be installed when a torque in the Z direction is required, and it is possible to perform levitation control and rotation control of the rotor 110 even when the side yoke 204a is not installed.

As described above, since the first coil 201a is configured to include the core 202a and the side yoke 204a, the magnetoresistance is reduced, and the magnetic flux density generated by the first permanent magnet 101a and the magnetic flux density generated by current flowing through the winding 203a can be increased. Since each magnetic flux density can be increased, the torque generated in each direction can also be increased.

Note that the second coil 201b has the same configuration as the first coil 201a. The core 202b, the winding 203b and the side yoke 204b, which are components of the second coil 201b, correspond to the core 202a, the winding 203a and the side yoke 204a, respectively, which are components of the first coil 201a.

The plurality of first permanent magnets 101a and the plurality of second permanent magnets 101b are preferably arranged in plane symmetry in the rotor 110 with the XY-plane as the plane of symmetry, that is, with a plane orthogonal to the Z-axis, which is the rotation axis, as the plane of symmetry. Further, it is preferable that the weight of the rotor 110 is equal in the Z direction. Since the weight of the rotor 110 is equal, the inclination and eccentricity of the rotor 110 caused by the variation of the weight can be reduced, so that the control of the rotor 110 can be easily performed. It is preferable that both the plurality of first coils 201a and the plurality of second coils 201b are arranged in plane symmetry in the stator 200 with the XY-plane as the plane of symmetry, that is, with a plane orthogonal to the Z-axis which is the rotation axis as the plane of symmetry. Thus, by arranging the set of the first permanent magnets 101a and the first coils 201a and the set of the second permanent magnets 101b and the second coils 201b symmetrically in this way, the positions where the attractive forces are generated in the respective sets are located in plane symmetry with the XY-plane as the plane of symmetry. Since the positions where the attractive forces are generated are located symmetrically in this way, the inclination and eccentricity of the rotor 110 caused by variations in the positions can be reduced, and the control of the rotor 110 can be easily performed.

Further, the plurality of first coils 201a and the plurality of second coils 201b are preferably arranged in plane symmetry in the stator 200 with the YZ-plane as the plane of symmetry, that is, with a plane including the Z-axis which is the rotation axis and oriented along the Y direction which is the direction of gravity as the plane of symmetry. The symmetry of such an arrangement makes the positions where the attractive forces are generated symmetrical with the YZ-plane as the plane of symmetry, which makes the control of the rotor 110 performed more easily.

Note that, although the first permanent magnets 101a and the second permanent magnets 101b, and the first coils 201a and the second coils 201b are arranged symmetrically in the above description, it is not necessary that they are arranged symmetrically. The inclination and eccentricity of the rotor 110 can be corrected by adjusting the gap between the coils and the permanent magnets and the amount of current flowing through the coils.

Here, using the first permanent magnets 101a and the first coils 201a as examples, the arrangement of these magnets and the thrust caused by them will be described with reference to FIG. 2A to FIG. 2D. Note that the arrangement and thrust of the second permanent magnets 101b and the second coils 201b are the same as those of the first permanent magnets 101a and the first coils 201a.

FIG. 2A is a transparent view of the arrangement of the plurality of first permanent magnets 101a viewed from the Y direction. In the present embodiment, as illustrated in FIG. 1B, twenty-four of first permanent magnets 101a are arranged. As illustrated in FIG. 2A, the twenty-four first permanent magnets 101a are arranged such that the first permanent magnets 101a-1 to the first permanent magnets 101a-24 are arranged at equal intervals in order to round the outer peripheral side surface of the rotor 110 in the Wz direction from the reference Or.

The first permanent magnets 101a are magnetized in the surface direction facing the first coils 201a. For example, the first permanent magnets 101a-1, 101a-3, 101a-19, and 101a-21 are magnetized to have an N-pole in the surface facing the first coil 201a, and the first permanent magnets 101a-18, 101a-22, and 101a-24 are magnetized to have an S-pole in the surface facing the first coil 201a. Further, for example, the first permanent magnets 101a-2, 101a-17, 101a-20, and 101-23 are divided into two portions in the Z direction, and are magnetized to have an N-pole and an S-pole in the respective portions.

Thus, the plurality of first permanent magnets 101a are arranged so that, on the side facing the first coils 201a, the magnetization units, in which the N-pole, the two-division magnetization, the N-pole, the S-pole, the two-division magnetization, and the S-pole are sequentially aligned in the Wz direction, are periodically aligned in the Wz direction.

Note that the plurality of first permanent magnets 101a may include a first permanent magnet line with different magnetic poles aligned in the Wz direction, which is the rotation direction, and a second permanent magnet line with different magnetic poles aligned in the Z direction, which is a direction intersecting the Wz. The Z direction is a direction along the Z-axis, which is the rotational axis. In FIG. 2A, the first permanent magnets 101a-1, 101a-3, 101a-18, 101a-19, 101a-21, 101a-22, and 101a-24 constitute the first permanent magnet line. In FIG. 2A, each of the first permanent magnets 101a-2, 101a-17, 101a-20, 101-23 constitutes the second permanent magnet line. The second permanent magnet line is not limited to the permanent magnet of two-division magnetization as illustrated in FIG. 2A, but may be composed of a plurality of permanent magnets. In this case, the plurality of permanent magnets constituting the second permanent magnet are arranged so that different magnetic poles are arranged in the Z direction, which is a direction intersecting the Wz. In the second permanent magnet line, different magnetic poles may be arranged not only in the Z direction but also in any direction intersecting with the Wz.

FIG. 2B is a transparent view of the arrangement of the first coils 201a viewed from the Y direction. In the present embodiment, as illustrated in FIG. 1B, twelve of the first coils 201a are arranged. As illustrated in FIG. 2B, the twelve first coils 201a are arranged so that the first coils 201a-1 to the first coils 201a-12 are sequentially aligned at equal intervals from the reference Oc in the Wz direction in an arc shape.

FIG. 2C and FIG. 2D are diagrams schematically illustrating the magnitudes of torques in a q-axis direction, a d-axis direction and the Z direction generated per unit current in the first coil 201a when the rotation angle $\theta$ of the rotor 110 is at $\theta 1$, that is, thrust constants Eq, Ed and Ez. The q-axis and the d-axis indicated here correspond to a q-axis and a d-axis in the motor control theory, respectively. FIG. 1B illustrates the directions of the q-axis and the d-axis with respect to the first coil 201a-10 as representative. The q-axis direction corresponds to the Wz direction, which is the circumferential direction, and the d-axis direction is the radial direction with the Z axis as the center. FIG. 2C illustrates the thrust constant Eq in the q-axis direction and the thrust constant Ed in the d-axis direction. FIG. 2D illustrates the thrust constant Ez in the Z direction.

As illustrated in FIG. 2C and FIG. 2D, the magnitude of each of the thrust constants Eq, Ed and Ez depends on the rotation angle $\theta 1$ of the rotor 110 and the index j of the first coil 201a. Here, the index j is a positive integer, and an integer satisfying $1 \leq j \leq 12$, for example. The first coil 201a can be identified by using the index j to denote "first coil 201a-j". The thrust constants Eq, Ed, and Ez can be expressed as Eq(j, $\theta$), Ed(j, $\theta$), and Ez(j, $\theta$), respectively, by displaying two arguments in parentheses, namely, the index j of the first coil 201a as the first argument and the rotation angle $\theta$ of the rotor 110 as the second argument. FIG. 2C and FIG. 2D illustrate the respective thrust constants when $\theta = \theta 1$.

In the case illustrated in FIG. 2A and FIG. 2B where $\theta = \theta 1$, the first coil 201a-10 is positioned substantially in the middle between the first permanent magnet 101a-24, whose facing face is an S-pole, and the first permanent magnet 101a-1, whose facing face is an N-pole. In this case, for example, a unit current is applied to the first coil 201a-10 so that an N-pole appears on the side facing the first permanent magnet 101a-24 and the first permanent magnet 101a-1. Note that the current is applied to the first coil 201a by a current control system 300 described later. When the unit current is applied to the first coil 201a-10 in this manner, an attractive force acts between the first coil 201a-10 and the first permanent magnet 101a-24, and a repulsive force acts between the first coil 201a-10 and the first permanent magnet 101a-1. That is, a thrust Eq(10, θ1) is applied to the first coil 201a-10 in the q-axis direction. On the other hand, a thrust Ed(10, θ1) applied to the first coil 201a-10 in the d-axis direction is relatively small.

Further, in the case illustrated in FIG. 2A and FIG. 2B, the first coil 201a-9 faces the first permanent magnet 101a-24, whose facing surface is an S-pole. In this case, for example, a unit current is applied to the first coil 201a-9 so that an S-pole appears on the side facing the first permanent magnet 101a-24. When the unit current is applied to the first coil 201a-9 in this manner, a repulsive force is applied between the first coil 201a-9 and the first permanent magnet 101a-24. That is, a thrust Ed(9, θ1) is applied to the first coil 201a-9 in the d-axis direction. On the other hand, a thrust Eq(9, θ1) applied to the first coil 201a-9 in the q-axis direction is relatively small.

Further, in the case illustrated in FIG. 2A and FIG. 2B, a unit current is applied to the first coil 201a-3 so that an N-pole appears on the side facing the first permanent magnet 101a-20. Then, since the first permanent magnet 101a-20 facing the first coil 201a-3 is magnetized by dividing it in the Z direction into an N-pole and an S-pole, a thrust Ez(3, θ1) in the Z direction acts on the first coil 201a-3.

The force acting on the first coil 201a can be treated as equivalent to the reaction force acting on the rotor 110. Therefore, for example, when thrust is generated in the positive direction in the q-axis direction in the first coil 201a, thrust is generated in the negative direction in the q-axis direction in the rotor 110. Thus, the force acting on the rotor 110 can be controlled by the first coil 201a and the first permanent magnet 101a. In accordance with the rotation angle θ1, the forces in the q-axis direction, the d-axis direction and the Z direction can be controlled by individually controlling the current applied to the coils using the respective thrust constants in the direction of control.

FIG. 3A is a side view of FIG. 1A viewed from the opposite side of FIG. 1B in the Z direction. As illustrated in FIG. 3A, the Z sensors 210a, 210b, and 210c are positioned laterally in the Z direction with respect to the rotor 110 so as to face the circumferential end face of the rotor 110 in the Z direction. The Z sensors 210a, 210b, and 210c are arranged at equal angular intervals in the Wz direction, for example. The Z sensors 210a, 210b, and 210c are arranged to detect the distance in the Z direction to the rotor 110, respectively.

FIG. 3B is a diagram for explaining a method of calculating the displacements of the rotor 110 in the Z direction, the Wx direction, and the Wy direction from the detection values of the Z sensors 210a, 210b, and 210c. In calculating each displacement, a plane ABC including points A, B and C is formed based on the detected values of the Z sensors 210a, 210b, and 210c. The X and Y coordinates of the point A are the X and Y coordinates of the Z sensor 210a, respectively, and the Z coordinate of the point A is a coordinate based on the detected value of the Z sensor 210a. The X and Y coordinates of the point B are the X and Y coordinates of the Z sensor 210b, respectively, and the Z coordinate of the point B is a coordinate based on the detected value of the Z sensor 210b. The X and Y coordinates of the point C are the X and Y coordinates of the Z sensor 210c, respectively, and the Z coordinate of the point C is a coordinate based on the detected value of the Z sensor 210c. The displacement in the Z direction, the displacement in the Wx direction, and the displacement in the Wy direction of the plane ABC can be calculated from the inclination of the normal vector of the plane ABC and the distance from the origin Os to the plane ABC. The displacement of the plane ABC in the Z direction, the displacement in the Wx direction and the displacement in the Wy direction can be treated as the displacement of the rotor 110 in the Z direction, the displacement of the rotor 110 in the Wx direction and the displacement the rotor 110 in the Wy direction, that is, the position of the rotor 110 in the Z direction, the rotation angle of the rotor 110 in the Wx direction and the rotation angle of the rotor 110 in the Wy direction, respectively. Thus, the displacements of the rotor 110 can be calculated using the plane ABC. Note that respective displacements are calculated by a motor controller 301 described below.

FIG. 4 is a connection diagram schematically illustrating a current control system 300 that controls a current applied to the first coils 201a and the second coils 201b. The current control system 300 will be described with reference to FIG. 4. The current control system 300 is a control unit that controls the drive of the rotor 110 by controlling the current applied to the plurality of first coils 201a and the plurality of second coils 201b. The configuration of the current control system 300 in connection with the first coils 201a will be described as an example, but the configuration in connection with the second coil 201b is also the same.

As illustrated in FIG. 4, the current control system 300 includes a motor controller 301, a plurality of current sensors 312a, and a plurality of current controllers 313a. The first coil 201a is individually connected to the current controller 313a. The current sensor 312a is individually connected to the current controller 313a. The current controller 313a can detect the current value of the first coil 201a by the current sensor 312a. Each of the current controllers 313a is connected to the motor controller 301.

The Z sensor 210a, 210b, and 210c, the Wz sensor 211, the X sensor 213, and the Y sensor 214 are connected to the motor controller 301. The motor controller 301 can detect the displacement X, Y, Z, Wx, Wy, and Wz of the rotor 110 based on the detection values of the respective sensors outputted from the Z sensors 210a, 210b, and 210c, the Wz sensor 211, the X sensor 213, and the Y sensor 214. The displacement X is a displacement in the X direction, the displacement Y is a displacement in the Y direction, the displacement Z is a displacement in the Z direction, the displacement Wx is a displacement in the Wx direction, the displacement Wy is a displacement in the Wy direction, and the displacement Wz is a displacement in the Wz direction. The motor controller 301 detects the displacement X based on the detection value of the X sensor 213, the displacement Y based on the detection value of the Y sensor 214, and the displacement Wz based on the detection value of the Wz sensor 211. The motor controller 301 detects the displacements Z, Wx, and Wy as described above based on the detection values of the Z sensors 210a, 210b, and 210c.

The motor controller 301 includes a control program, clock, and the like inside it, and calculates the current value to be applied to each first coil 201a according to each displacement of the rotor 110. The motor controller 301 transmits a current command value to each current controller 313a, which commands the calculated current value to be applied to each first coil 201a.

The current controller 313a individually applies a predetermined current to the corresponding first coil 201a while detecting the current amount by the current sensor 312a according to the current command value from the motor controller 301.

FIG. 5 schematically illustrates a control loop for calculating the magnitude of the torque applied to the rotor 110. A method of controlling the attitude of the rotor 110 by the motor controller 301 will be described with reference to FIG. 5. In FIG. 5, ref is a target value of the displacement of the rotor 110. Also, pos is the displacement of the rotor 110 acquired from the detection value of the sensor group (the Z sensor 210a, 210b, and 210c, the Wz sensor 211, the X sensor 213, and the Y sensor 214). The motor controller 301 includes an attitude controller 501 and a current calculator 502.

As illustrated in FIG. 5, the attitude controller 501 calculates a torque T applied to the rotor 110 from the difference err between the target value ref and the displacement pos. The attitude controller 501 may be, for example, a controller by PID control or a controller using a filter as appropriate according to the characteristics of the rotor 110. Such a controller can stabilize the attitude of the rotor 110.

The current calculator 502 determines the current I applied to the first coil 201a and the second coil 201b from the torque T and the displacement pos. The current controllers 313a and 313b apply the determined current I to the first coil 201a and the second coil 201b, respectively.

When a current I is applied to the first coil 201a and the second coil 201b, an electromagnetic force F is generated between the first coil 201a and the second coil 201b and the rotor 1 10 to act on the rotor 110. Thus, levitation control and rotation control of the rotor 110 are performed. The attitude controller 501 detects displacement pos again from the detection values of the Z sensors 210a, 210b, and 210c, the Wz sensor 211, the X sensor 213, and the Y sensor 214, and repeats the above processing. In this way, the levitation control and the rotation control of the rotor 110 are performed to control the drive of the rotor 110.

The torque vector Tq indicating the torque T applied to the rotor 110 as described above is expressed by the following Expression (1). Tx, Ty, and Tz are the magnitudes of the torque in the X direction, the torque in the Y direction, and the torque in the Z direction, respectively. Twx, Twy, and Twz are the magnitudes of the moments of force around the X-axis, the Y-axis, and the Z-axis, respectively. The torque here includes the force and the moment of force, and the torque along an axis such as in the X direction, the Y direction, the Z direction, or the like is the force.

$$Tq = (Tx, Ty, Tz, Twx, Twy, Twz) \qquad ... \text{Expression (1)}$$

The rotation drive apparatus 10 according to the present embodiment controls the current flowing through the first coils 201a and the second coils 201b by the current control system 300 to control the respective components Tx, Ty, Tz, Twx, Twy, and Twz of the torque vector Tq. Thus, the rotation drive apparatus 10 can rotate the rotor 110 in the Wz direction while controlling the attitude (X, Y, Z, Wx, Wy) of the rotor 110. Note that, in the attitude of the rotor 110, X, Y and Z are positions in the X direction, the Y direction and the Z direction, respectively, and Wx and Wy are rotation angles around the X-axis and the Y-axis, respectively.

FIG. 6 is a diagram for explaining torques generated in the q-axis direction, the d-axis direction and the Z direction at an arbitrary position of the rotor 110. Here, FIG. 6 is used to explain that the torque vector Tq can be applied to the rotor 110 at an arbitrary rotation angle in the Wz direction of the rotor 110 by moving a magnetic field generated in the first coils 201a and the second coils 201b in accordance with the rotation of the rotor 110.

In the present embodiment, since the first coils 201a and the second coils 201b are arranged in the stator 200, torques can be generated in the q-axis direction, the d-axis direction and the Z direction. As illustrated in FIG. 2A, each of the plurality of first permanent magnets 101a and the plurality of second permanent magnets 101b constitute a permanent magnet line including permanent magnets divided in the Z direction and permanent magnets not divided in the Z direction, respectively. In the permanent magnet line, the permanent magnets not divided in the Z direction mainly contribute to the torque in the q-axis direction and the d-axis direction, and the permanent magnets divided in the Z direction mainly contribute to the torque in the Z direction.

Here, symbols used for the explanation are summarized. In FIG. 6, the symbols are shown as appropriate.

j: index for distinguishing the coils in the first coils 201a or the second coils 201b (j = 1 to 12)

Ij: current value applied to the j-th first coil 201a-j or the j-th second coil 201b-j

Φj: angle in the Wz direction of the j-th first coil 201a-j or the j-th second coil 201b-j

r: radius to the first permanent magnet 101a or the second permanent magnet 101b

*: multiplication symbol

Σ: sum when the index j is changed from 1 to 12

Symbols for forces acting on the first coil 201a are as follows. Eqj, Edj and Ezj shown in FIG. 6 correspond to Eqa(j, 0), Eda(j, θ) and Eza(j, θ) shown below, respectively.

Eqa(j, θ): force per unit current in the q-axis direction acting between the j-th first coil 201a-j and the rotor 110 at the rotation angle θ

Eda(j, θ): force per unit current in the d-axis direction acting between the j-th first coil 201a-j and the rotor 110 at the rotation angle θ

Eza(j, θ): force per unit current in the Z direction acting between the j-th first coil 201a-j and the rotor 110 at rotation angle θ

Symbols for forces acting on the second coil 201b are as follows. Eqj, Edj and Ezj shown in FIG. 6 correspond to Eqb(j, 0), Edb(j, θ) and Ezb(j, θ) shown below, respectively.

Eqb(j, θ): force per unit current in the q-axis direction acting between the j-th second coil 201b-j and the rotor 110 at the rotation angle θ

Edb(j, θ): force per unit current in the d-axis direction acting between the j-th second coil 201b-j and the rotor 110 at the rotation angle θ

Ezb(j, θ): force per unit current in the Z direction acting between the j-th second coil 201b-j and the rotor 110 at the rotation angle θ

The torque vector Tq generated in the rotor 110 is represented by Expression (1) shown above. The components of the torque vector generated by the first coils 201a are represented by Txa, Tya, Tza, and Twza, and the components of the torque vector generated by the second coils 201b are represented by Txb, Tyb, Tzb, and Twzb. These components are components in the following directions.

Txa: component in the X direction of the torque vector generated by the first coils 201a

Tya: component in the Y direction of the torque vector generated by the first coils 201a

Tza: component in the Z direction of the torque vector generated by the first coils 201a

Twza: component in the Wz direction of the torque vector generated by the first coils 201a

Txb: component in the X direction of the torque vector generated by the second coils 201b

Tyb: component in the Y direction of the torque vector generated by the second coils 201b

Tzb: component in the Z direction of the torque vector generated by the second coils 201b

Twzb: component in the Wz direction of the torque vector generated by the second coils 201b

The components Txa, Tya, Tza, and Twza are represented by the following Expressions (2-1) to (2-4), respectively.

$$\mathrm{Txa} = \Sigma\{(-\mathrm{Eqa}(j, \theta) * \sin\Phi j + \mathrm{Eda}(j, \theta) * \cos\Phi j) * \mathrm{Ij}\} \ ... \ \text{Expression (2-1)}$$

$$\mathrm{Tya} = \Sigma\{(\mathrm{Eqa}(j, \theta) * \cos\Phi j + \mathrm{Eda}(j, \theta) * \sin\Phi j) * \mathrm{Ij}\} \ ... \ \text{Expression (2-2)}$$

$$\mathrm{Tza} = \Sigma\{\mathrm{Eza}(j, \theta) * \mathrm{Ij}\} \qquad ... \ \text{Expression (2-3)}$$

$$\mathrm{Twza} = \Sigma\{\mathrm{Eqa}(j, \theta) * r * \mathrm{Ij}\} \qquad ... \ \text{Expression (2-4)}$$

The components Txb, Tyb, Tzb, and Twzb are represented by the following Expressions (2-5) to (2-8), respectively.

$$Txb = \Sigma\{(-Eqb(j, \theta) * \sin\Phi j + Edb(j, \theta) * \cos\Phi j)*Ij\} \quad ... \text{ Expression (2-5)}$$

$$Tyb = \Sigma\{(Eqb(j, \theta) * \cos\Phi j + Edb(j, \theta)* \sin\Phi j) * Ij\} \quad ... \text{ Expression (2-6)}$$

$$Tzb = \Sigma\{Ezb(j, \theta) * Ij\} \qquad ... \text{ Expression (2-7)}$$

$$Twzb = \Sigma\{Eqb(j, \theta) * r * Ij\} \qquad ... \text{ Expression (2-8)}$$

Here, as illustrated in FIG. 1C, it is assumed that the first coils 201a and the second coils 201b are arranged at a distance t from the XY-plane in the Z direction in plane symmetry with the XY-plane as the plane of symmetry. Then, the components Tx, Ty, Tz, Twx, Twy, and Twz of the torque vector Tq are represented by the following Expressions (3-1) to (3-6), respectively.

$$Tx = Txa + Txb \qquad ... \text{ Expression (3-1)}$$

$$Ty = Tya + Tyb \qquad ... \text{ Expression (3-2)}$$

$$Tz = Tza + Tzb \qquad ... \text{ Expression (3-3)}$$

$$Twx = (Tya - Tyb) * t \qquad ... \text{ Expression (3-4)}$$

$$Twy = (Txa - Txb) * t \qquad ... \text{ Expression (3-5)}$$

$$Twz = Twza + Twzb \qquad ... \text{ Expression (3-6)}$$

To apply the desired torque vector Tq, the current Ij satisfying the above Expressions (2-1) to (2-8) and (3-1) to (3-6) may be applied to each of the first coils 201a and the second coils 201b. The current control system 300 can control the application of the current Ij as such. Note that each coil generates some torque in the d-axis direction. The torque in the d-axis direction generated by each coil can be cancelled out by arranging the first coils 201a and the second coils 201b in plane symmetry with the XY-plane and the YZ-plane as the planes of symmetrical, because the directions of the torques are opposite to each other.

In addition to the control described above, the currents applied to the coils can be further controlled by the current control system 300 to further apply the force of Twy to the rotor 110. Specifically, the plurality of first coils 201a are arranged on the upstream side and the downstream side with respect to the YZ-plane, and the currents of the first coils 201a facing the first permanent magnets 101a divided in the Z direction can be controlled so as to generate opposite torques on the upstream side and the downstream side.

For example, in FIG. 1C, FIG. 2A, and FIG. 2B, the first coils 201a-1 to 201a-6 are arranged on the upstream side and the first coils 201a-7 to 201a-12 are arranged on the downstream side. The first permanent magnets 101a-20 and 101a-23 divided in the Z direction are positioned so as to face the first coils 201a-3 and 201a-8, respectively. Note that the relationship between the second coils 201b and the second permanent magnets 101b is the same. In this case, in order to obtain the torque in the Z direction, the currents are applied to the first coils 201a facing the first permanent magnets 101a divided in the Z direction. When both the first coil 201a-3 on the upstream side and the first coil 201a-8 on the downstream side are applied with the currents in the same direction, the torque in the Z direction can be obtained as shown in Expression (3-3) above. On the other hand, for example, when the first coil 201a-3 on the upstream side is applied with the current in the positive direction, and the first coil 201a-8 on the downstream side is applied with the current in the negative direction, the torques generated in the Z direction are opposite on the upstream side and the downstream side, and thus the torque in the Wy direction can be obtained. Note that, since the first coil 201a-12 also faces the first permanent magnet 101a-2 divided in the Z direction, the torque may be obtained by adding the first permanent magnet 101a-2 in the same manner.

Note that FIG. 2A and FIG. 2B illustrate a case where the first permanent magnets 101a-2, 101a-20, and 101a-23 divided in the Z direction, which is the second permanent magnet line, face the first coils 201a-1 to 201a-12. When the rotor 110 rotates in the Wz direction, the first permanent magnets 101a also rotate in the Wz direction. The torque Tz in the Z direction is controlled by the currents flowing through the first coils 201a facing the first permanent magnets 101a divided in the Z direction. Therefore, when the rotor 110 rotates in the Wz direction, it is desirable that the plurality of first permanent magnets 101a are arranged so that at least one or more of the first permanent magnets 101a divided in at least the Z direction face the plurality of first coils 201a arranged in an arc shape. The relative positional relationship between the first coils 201a and the first permanent magnets 101a makes it possible to always obtain the torque Tz in the Z direction. That is, when the first permanent magnets 101a divided in the Z direction face one or more of the first coils 201a-1 to 201a-12, the torque Tz in the Z direction can always be obtained regardless of the rotation angle $\theta$ of the rotor 110. Thus, the torque Tz in the Z direction can always be obtained during rotation of the rotor 110, so that stable rotation control of the rotor 110 can be performed. Note that the relative positional relationship between the second coils 201b and the second permanent magnets 101b can be made similar to the relative positional relationship between the first coils 201a and the first permanent magnets 101a described above.

Here, the distances from the YZ-plane to the points where the first permanent magnets 101a are divided in the Z direction on the upstream side and the downstream side are h1 and h2, respectively. Note that the distances h1 and h2 change with the rotation when the rotor 110 is rotating.

The torque Tza(j = 1 to 6) in the Z direction generated by the first coil 201a-1 to 201a-6 on the upstream side and the torque Tzb in the Z direction generated by the second coil 201b-1 to 201b-6 on the upstream side are obtained by the following Expressions (4-1) and (4-2), respectively. In the Expressions (4-1) and (4-2), $\Sigma$ is the sum when the index j is changed from 1 to 6.

$$Tza(j = 1 \text{ to } 6) = \Sigma\{Eza(j, \theta) * Ij\} \qquad \text{... Expression (4-1)}$$

$$Tzb(j = 1 \text{ to } 6) = \Sigma\{Ezb(j, \theta) * Ij\} \qquad \text{... Expression (4-2)}$$

The torque Tza(j = 7 to 12) in the Z direction generated by the first coils 201a-7 to 201a-12 on the downstream side is obtained by the following Expression (4-3). The torque Tzb(j = 7 to 12) in the Z direction generated by the second coil 201b-7 to 201b-12 on the downstream side is obtained by the following Expression (4-4). In the Expressions (4-3) and (4-4), $\Sigma$ is the sum when the index j is changed from 7 to 12.

$$Tza(j = 7 \text{ to } 12) = \Sigma\{Eza(j, \theta) * Ij\} \qquad \text{... Expression (4-3)}$$

$$Tzb(j = 7 \text{ to } 12) = \Sigma\{Ezb(j, \theta) * Ij\} \qquad \text{... Expression (4-4)}$$

Then, the torque Twy in the Wy direction represented by the following Expression (4-5) is obtained by Expression (3-5) and Expressions (4-1) to (4-4).

$$Twy = (Txa - Txb) * t + \{(Tza(j = 1 \text{ to } 6) + Tzb(j = 1 \text{ to } 6)) * h1 - (Tza(j = 7 \text{ to } 12) + Tzb(j = 7 \text{ to } 12)) * h2\} \qquad \text{Expression (4-5)}$$

Thus, the rotation drive apparatus 10 according to the present embodiment can control the rotational speed and attitude of the rotor 110 because the current control system 300 can apply the torque vector Tq having the components of six axes to the rotor 110 as described above.

FIG. 7A and FIG. 7B are schematic diagrams illustrating the attractive force acting between the first permanent magnet 101a of the rotor 110 and the core 202a of the first coil 201a. The attractive force acting between the core 202a of the first coil 201a and the first permanent magnet 101a will be described with reference to FIG. 7A and FIG. 7B. Note that the second coil 201b and the second permanent magnet 101b have the same structure, and the same attractive force acts between them.

The core 202a arranged within the first coil 201a is made of a magnetic material. Therefore, as illustrated in FIG. 7A, an attractive force fmar, which is a magnetic force, is generated between the core 202a and the first permanent magnet 101a. The attractive force fmar is decomposed into an attractive force fmax in the X-axis direction and an attractive force fmay in the Y-axis direction. The magnitude and direction of the attractive force fmar depend on the relative positional relationship between the core 202a and the first permanent magnet 101a, as illustrated by arrows in FIG. 7B.

According to the configuration of the present embodiment, in the Y direction which is the direction of gravity, the combined force obtained by adding the attractive force fmay acting on the first coils 201a-1 to 201a-12 in the Y direction becomes the attractive force Fmay generated in the direction of gravity of the rotor 110.

On the other hand, in the X direction, since the first coils 201a-1 to 201a-6 and the first coils 201a-7 to 201a-12 are arranged in plane symmetry with the YZ-plane as the plane of symmetry, attractive force is also generated in plane symmetry with the YZ-plane as the plane of symmetry. Specifically, in the X direction, the attractive forces act in the positive direction in the first coils 201a-1 to 201a-6 and in the negative direction in the first coils 201a-7 to 201a-12. When the rotor 110 is centered in the X direction, the attractive forces in the positive direction and the attractive forces in the negative direction in the X direction cancel each other. That is, in this case, the combined force of the attractive forces fmax in the X direction acting on the first coils 201a-1 to 201a-6 and the combined force of the attractive forces fmax in the X direction acting on the first coils 201a-7 to 201a-12 have approximately the same magnitudes to cancel out each other.

In the present embodiment, as described above, the first coils 201a have the cores 202a, which causes the attractive force Fmay to act on the rotor 110 in the Y direction. Also, the second coils 201b have the cores 202b, which causes the attractive force Fmby to act on the rotor 110 in the Y direction as well as the attractive force Fmay. The attractive forces Fmay and Fmby in the Y direction are used as a force for levitating the rotor 110 in the Y direction. Note that the first coil 201a and the second coil 201b are not necessarily limited to a cored coil having a core. For example, the first coil 201a and the second coil 201b may be an air-core coil when the weight of the rotor 110 is light enough or depending on the positioning accuracy required for the rotor 110. In the case of the air-core coil, the torque Ty in the Y direction is used as a force for levitating the rotor 110 in the Y direction.

FIG. 8A, FIG. 8B, and FIG. 8C are schematic diagrams of the movement of the rotor 110 in the Y direction with the center position of the rotor 110 in the X direction aligned with the origin Os. The levitation of the rotor 110 will be described with reference to FIG. 8A to FIG. 8C.

The rotor 110 is arranged at a position where its center O overlaps with the origin Os. When the rotor 110 is moved in the Y direction, there is an equilibrium point ep at which the magnitude of the gravity force Fg of the rotor 110 is equal to the magnitude of the attractive forces Fmay + Fmby. Note that, in the present embodiment, the origin Os and the equilibrium point ep are configured to overlap with each other. FIG. 8A illustrates a state in which the rotor 110 is arranged so that the center O of the rotor 110 overlaps with the equilibrium point ep.

For example, when the position of the rotor 110 is shifted in the positive direction in the Y direction as illustrated in FIG. 8B from the state illustrated in FIG. 8A, the attractive force Fmay + Fmby becomes larger than the gravity force Fg. On the other hand, the position of the rotor 110 is shifted in the negative direction in the Y direction as illustrated in FIG. 8C from the state illustrated in FIG. 8A, the gravity force Fg becomes larger than the attractive force Fmay + Fmby. The rotor 110 moves in the direction of the larger force, that is, in the direction of the attractive force Fmay + Fmby in the case illustrated in FIG. 8B, and in the direction of the gravity force Fg in the case illustrated in FIG. 8C.

Therefore, the rotation drive apparatus 10 applies currents to the first coils 201a and the second coils 201b by the current control system 300 at a position where the center O of the rotor 110 overlaps with the equilibrium point ep to control the torque in the Y direction applied to the rotor 110. Further, the rotation drive apparatus 10 also controls the torque applied to the rotor 110 in the X direction in the same manner to maintain the levitation of the rotor 110.

In order to maintain the levitation of the rotor 110, it is desirable to control the torque applied to the rotor 110 at the position where the center O of the rotor 110 overlaps the equilibrium point ep. When the control is performed at the equilibrium point ep in this way, the levitation of the rotor 110 can be continued at smaller currents than the currents flowing through the first coils 201a and the second coils 201b. Further, by applying currents to the first coils 201a and the second coils 201b so as to generate the torque in the Wz direction as described above, the rotor 110 can rotate in the Wz direction while keeping the rotor 110 levitated so that the center O of the rotor 110 overlaps the equilibrium point ep. Thus, the rotation drive apparatus 10 can rotate the rotor 110 in the Wz direction while levitating the rotor 110 in the Y direction by controlling the current flowing through the first coils 201a and the second coils 201b by the current control system 300 to control the coils with respect to the rotor 110.

Note that, in order to position the rotor 110 at a position where the center O of the rotor 110 overlaps the equilibrium point ep, the rotor 110 can be lifted and positioned using a general elevator. Further, the rotor 110 can also be lifted and positioned by controlling the torque Ty in the Y direction by applying a current to the first coils 201a and the second coils 201b.

As described above, according to the present embodiment, the levitation control and the rotation control of the rotor 110 can be performed with a simple configuration without requiring numerous actuators. Therefore, according to the present embodiment, the rotor 110 can be levitated to perform the levitation control and the rotation control of the rotor 110 while avoiding enlargement of the whole rotation drive apparatus 10.

[Second Embodiment]

A rotation drive apparatus according to a second embodiment of the present invention will be described with reference to FIG. 9A to FIG. 9C. FIG. 9A is a perspective view illustrating the rotation drive apparatus 10 according to the present embodiment. FIG. 9B is a side view of the rotation drive apparatus 10 according to the present embodiment viewed in the Z direction. FIG. 9C is a cross-sectional view taken along a line B-B' illustrated in FIG. 9B viewed in the X direction. Note that the same components as those in the above first embodiment are labeled with the same references, and the description thereof will be omitted or simplified.

In addition to the first coil 201a and the second coil 201b arranged outside the rotor 110 as in the first embodiment, the rotation drive apparatus 10 according to the present embodiment includes the first coils 201a and the second coils 201b arranged inside the rotor 110. In addition, the rotation drive apparatus 10 according to the present embodiment includes the first permanent magnets 101a and the second permanent magnets 101b arranged on the inner peripheral side surface of the rotor 110 in addition to the first permanent magnets 101a and the second permanent magnets 101b arranged on the outer peripheral side surface of the rotor 110.

As illustrated in FIG. 9A to FIG. 9C, the stator 200 is provided with the first coils 201a-1 to 201a-12 as a coil group outside the rotor 110 in the same manner as in the first embodiment. Further, the stator 200 is provided with a plurality of first coils 201a-51 to 201a-62 as another coil group inside the rotor 110. The plurality of first coils 201a-51 to 201a-62 are arranged so as to be faceable to the plurality of first permanent magnets 101a installed so as to be arranged circumferentially in one line uniformly along the Wz direction, which is the rotational direction, on the circumferential inner surface of the rotor 110. The plurality of first coils 201a-51 to 201a-62 are mounted so as to be uniformly arranged in one line in an arc shape along the Wz direction. The plurality of first coils 201a-51 to 201a-62 are arranged in plane symmetry with the YZ-plane as the plane of symmetry. Note that the second coils 201b are also arranged inside the rotor 110 in the same manner as the first coil 201a.

In the cylindrical rotor 110, the plurality of first permanent magnets 101a are uniformly arranged as a permanent magnet group in the circumferential direction on the outer peripheral side surface of the rotor 110 so as to face the first coils 201a-1 to 201a-12 in the same manner as in the first embodiment. Further, the plurality of first permanent magnets 101a are installed as another permanent magnet group in the rotor 110 so as to be uniformly arranged in one line in an arc shape along the Wz direction on the inner peripheral side of the rotor 110 so as to be faceable to the first coils 201a-51 to 62 arranged inside the rotor 110. Note that, as illustrated in FIG. 9C, the first permanent magnets 101a may be arranged in holes formed in the base member of the rotor 110 including the yoke 102 so as to penetrate between the outer peripheral side and the inner peripheral side. In this case, the first permanent magnets 101a face the outer peripheral surface and the inner peripheral surface of the rotor 110, with the faces magnetized in opposite polarity to each other. In this way, the first permanent magnet 101a can also be used with respect to the first coils 201a-1 to 201a-12 installed on the outside of the rotor 110 and the first coils 201a-51 to 201a-62 installed on the inside. Note that the second permanent magnets 101b are also installed on the inner peripheral side surface of the rotor 110 in the same manner as the first permanent magnets 101a.

In the present embodiment, as in the first embodiment, attractive forces act between the first coils 201a-1 to 201a-12 and the first permanent magnets 101a, which are installed outside the rotor 110. Further, in the present embodiment, attractive forces act between the first coils 201a-51 to 201a-62 and the first permanent magnets 101a, which are installed inside the rotor 110. In addition, the torque in each direction can be controlled by applying currents to the first coils 201a-1 to 201a-12 installed outside and the first coils 201a-51 to 201a-62 installed inside.

According to the present embodiment, since the attractive force can be increased, the rotor 110 can be easily levitated even when the weight of the rotor 110 is heavy. Also, according to the present embodiment, the torque required for acceleration when the rotor 110 is rotated can be further increased. Further, by controlling the torque in each direction by the first coils 201a-1 to 201a-12 installed outside and the first coils 201a-51 to 201a-62 installed inside, it is possible to reduce the shake of the rotor 110 when rotating due to disturbance or the like. Therefore, according to the present embodiment, the levitation control and the rotation control of the rotor 110 can be performed more stably.

[Third Embodiment]

A rotation drive apparatus according to a third embodiment of the present invention will be described with reference to FIG. 10A to FIG. 10D. FIG. 10A is a perspective view illustrating the rotation drive apparatus 10 according to the present embodiment. FIG. 10B is a side view of the rotation drive apparatus 10 according to the present embodiment viewed in the Z direction. FIG. 10C is a top view of the rotation drive apparatus 10 according to the present embodiment viewed in the Y direction. FIG. 10D is a cross-sectional view illustrating taken along C-C' illustrated in FIG. 10B. In FIG. 10B, the side yoke 204a is omitted for ease of explanation. Note that the same components as those in the above first and second embodiments are labeled with the same references, and the description thereof will be omitted or simplified.

In the rotation drive apparatus 10 according to the present embodiment, the plurality of first coils 201a and the plurality of second coils 201b are installed in arc shapes outside the rotor 110 as in the first embodiment, and are further installed outside the rotor 110 in the circumferential direction all around the circumference. Note that the configuration of the first coils 201a and the first permanent magnets 101a will be described below as an example, but the second coils 201b and the second permanent magnets 101b have the same configuration.

As illustrated in FIG. 10A to FIG. 10D, the stator 200 is provided with a plurality of first coils 201a-1 to 201a-36 arranged in a line in order in the circumferential direction outside the rotor 110 to go around the rotor 110. The first coils 201a-1 to 201a-12 are a coil group arranged in an arc shape along the Wz direction, which is the rotation direction, as in the first embodiment. In the present embodiment, the first coils 201a-13 to 201a-36, which are another coil group, are arranged in an arc shape along the Wz direction in the remaining arc portion on the circumference including the arc portion lined with the first coils 201a-1 to 201a-12.

The first coils 201a includes two kinds of coils, cored coils and air-core coils. The first coils 201a-1 to 201a-12 are cored coils as in the first embodiment. On the other hand, the first coil 201a-13 to 201a-36 are air-core coils. The respective configurations will be described using the first coils 201a-5 and 201a-23 as examples.

As illustrated in FIG. 10D, the first coil 201a-5, which is the cored coil, includes the core 202a-5, the winding 203a-5, and the side yoke 204a-5 as in the first embodiment. On the other hand, the first coil 201a-23, which is the air-core coil, includes a winding 203a-23 and a side yoke 204a-23, and has no core. Note that the configuration of the first coil 201a-23, is the same as that of the first coil 201a-5, except that the first coil 201a-23 does not have any core.

Thus, the first coils 201a-1 to 201a-12 are the cored coils including the cores 202a-1 to 202a-12, respectively. Since the first coils 201a-1 to 201a-12 include the cores 202a-1 to 202a-12, attractive forces act between the cores 202a-1 to 201a-12 and the first permanent magnets 101a.

On the other hand, the first coils 201a-13 to 201a-36 are the air-core coil without any core. Since the first coils 201a-13 to 201a-36 do not include any core, no attractive force acts between the first coils 201a-13 to 201a-36 and the first **permanent** magnets 101a. Note that the first coils 201a-13 to 201a-36 do not necessarily need to be air-core coils. For example, when the weight of the rotor 110 is sufficiently light or depending on the positional accuracy required for the rotor 110, the first coils 201a-13 to 201a-36 may be cored coils.

In the present embodiment, as in the first embodiment, the equilibrium point ep at which the gravity force Fg of the rotor 110 and the magnitude of the attractive forces Fmay + Fmby are equal can be set as the rotation center of the rotor 110. The attractive forces Fmay + Fmby is the sum of the attractive force Fmay between the first coils 201a and the first permanent magnets 101a and the attractive force Fmby between the second coils 201b and the second permanent magnets 101b.

Also in the present embodiment, as in the first embodiment, the current control system 300 allows the first coils 201a-1 to 201a-12 and the second coils 201b-1 to 201b-12 to be applied with current to thereby obtain torque in each direction acting on the rotor 110.

On the other hand, the torque in each direction acting on the rotor 110 can also be obtained by applying current to the first coils 201a-13 to 201a-36 by the current control system 300. However, since the first coils 201a-13 to 201a-36 do not include any core, the torque obtained by applying current to the first coils 201a-13 to 201a-36 is smaller than that of the first coils 201a-1 to 12. Note that the second coils 201b-13 to 201b-36, which are the same as the first coils 201a-13 to 201a-36, are also applied with current in the same manner, thereby obtaining torque in each direction acting on the rotor 110.

In the present embodiment, the levitation control and the rotation control of the rotor 110 can be performed by adding the torque obtained by applying current to the first coils 201a-13 to 201a-36 and the second coils 201b-1 to 201b-36. Thereby, the position and the range in which the torque can be controlled in the circumferential direction of the rotor 110 can be expanded, and as a result, the rotation of the rotor 110 can be made more stable.

Note that, in the present embodiment, the explanation is given using a case where the first coils 201a and the second coils 201b are arranged over the entire circumference of the rotor 110 as an example, but they do not necessarily have to be arranged over the entire circumference. FIG. 11A and FIG. 11B are side views of rotation drive apparatuses 10 according to modifications of the present embodiment as viewed in the Z direction. The first coils 201a and the second coils 201b may be arranged as illustrated in FIG. 11A or as illustrated in FIG. 11B.

In the case illustrated in FIG. 11A, the first coils 201a-1 to 201a-12 are arranged in an arc shape on the upper side of the rotor 110 as in the first embodiment. Further, in the case illustrated in FIG. 11A, the first coils 201a-19 to 201a-30 are arranged in an arc shape on the lower side of the rotor 110. In this case also, the first coils 201a-1 to 201a-12 may be cored coils and the first coils 201a-19 to 201a-30 may be air-core coils. The first coils 201a-19 to 201a-30 are preferably arranged in plane symmetry with the YZ-plane as the plane of symmetry. The second coils 201b may also be arranged in the same manner as the first coils 201a illustrated in FIG. 11A.

In the case illustrated in FIG. 11B, in addition to the first coils 201a-1 to 201a-12 similar to FIG. 11A, the first coils 201a-16 to 201a-21 and the first coils 201a-26 to 201a-32 are arranged on the lower side of the rotor 110. The first coils 201a-16 to 201a-21 and the first coils 201a-26 to 201a-32 are circumferentially spaced and arranged in an arc shape. The first coils 201a-16 to 201a-21 are arranged on one side in the X direction on the lower side of the rotor 110, and the first coils 201a-26 to 201a-32 are arranged on the other side in the X direction on the lower side of the rotor 110. In this case also, the first coils 201a-1 to 201a-12 may be cored coils, and the first coils 201a-16 to 201a-21 and the first coils 201a-26 to 201a-32 may be air-core coils. The first coils 201a-16 to 201a-21 and the first coils 201a-26 to 201a-32 are preferably arranged in plane symmetry with the YZ-plane as the plane of symmetry. The second coils 201b may also be arranged in the same manner as the first coils 201a illustrated in FIG. 11B.

As illustrated in FIG. 11A and FIG. 11B, the first coil 201a and the second coil 201b may be partially arranged in the circumferential direction of the rotor 110. By partially arranging the first coils 201a and the second coils 201b, stable rotation control of the rotor 110 can be performed while keeping the cost low.

The configuration of the present embodiment can also be combined with the configuration of the second embodiment. That is, the first coils 201a and the second coils 201b may be installed inside the rotor 110 in the rotation drive apparatus 10 according to the present embodiment as in the second embodiment.

[Fourth Embodiment]

A rotation drive apparatus according to a fourth embodiment of the present invention will be described with reference to FIG. 12A to FIG. 16. Note that the same components as those in the above first to third embodiments are labeled with the same references, and the description thereof will be omitted or simplified.

First, the configuration of the rotation drive apparatus according to the present embodiment will be described with reference to FIG. 12A to FIG. 12E. FIG. 12A is a perspective view illustrating the rotation drive apparatus 10 according to the present embodiment. FIG. 12B and FIG. 12C are side views of the rotation drive apparatus 10 according to the present embodiment viewed in the Z direction, respectively. FIG. 12D is a top view of the rotation drive apparatus 10 according to the present embodiment viewed in the Y direction. FIG. 12E is a cross-sectional view taken along a line D-D' illustrated in FIG. 12B and FIG. 12C.

In the rotation drive apparatus 10 according to the present embodiment, the arrangement of the first permanent magnets 101a and the second permanent magnets 101b in the rotor 110 and the arrangement of the first coils 201a and the second coils 201b in the stator 200 are different from those in the first embodiment.

As illustrated in FIG. 12A, the plurality of first permanent magnets 101a are mounted on the circumferential end face of the cylindrical rotor 110, which is the end face of one side in the Z direction, so as to be uniformly arranged in one line in a circumferential shape along the Wz direction, which is the rotational direction. Like the plurality of first permanent magnets 101a, the plurality of second permanent magnets 101b are mounted on the circumferential end face of the rotor 110, which is the end face of the other side in the Z direction, so as to be uniformly arranged in one line in a circumferential shape along the Wz direction. Thus, the plurality of first permanent magnets 101a and the plurality of second permanent magnets 101b are arranged in the rotor 110.

The plurality of first coils 201a and the plurality of second coils 201b are arranged in the stator 200 so as to face the first permanent magnets 101a and the second permanent magnets 101b on one side and the other side in the Z direction of the rotor 110 outside the rotor 110. The plurality of first coils 201a are mounted in a position where the plurality of first coils 201a can face the first permanent magnets 101a so as to be arranged uniformly in one line in a circumferential shape along the Wz direction. The plurality of second coils 201b are mounted in a position where the plurality of second coils 201b can face the second permanent magnets 101b so as to be arranged uniformly in one line in a circumferential shape along the Wz direction. Thus, the plurality of first coils 201a and the plurality of second coils 201b are arranged in the stator 200.

The set of the first coils 201a and the first permanent magnets 101a, and the set of the second coils 201b and the second permanent magnets 101b are arranged on the stator 200 and the rotor 110 in plane symmetry with the XY-plane as the plane of symmetry. Thus, the rotor 110 is arranged so as to be sandwiched between the plurality of first coils 201a and the plurality of second coils 201b from both sides, and rotates around the Z-axis by rotation control while levitating in the Y direction by levitation control.

In the present embodiment, the first coils 201a-1 to 201a-36 are arranged in order in the Θ direction with the reference Oc as the reference of the stator 200. Similarly, the second coils 201b-1 to 201b-36 are arranged in order in the Θ direction with the reference Oc as the reference of the stator 200. Further, the first permanent magnets 101a-1 to 101a-24 are arranged in order in the Θ direction with the reference Or as the reference of the rotor 110. Similarly, the second permanent magnets 101b-1 to 101b-24 are arranged in order in the Θ direction with the reference Or as the reference of the rotor 110.

Note that the numbers of the first coils 201a, the second coils 201b, the first permanent magnets 101a, and the second permanent magnets 101b are not limited to the numbers illustrated in the present embodiment, respectively. The numbers of the first coils 201a, the second coils 201b, the first permanent magnets 101a, and the second permanent magnets 101b can be suitably changed according to the thrust required in the q-axis direction, the d-axis direction, and the R-axis direction, which will be described later, and the like. Although the number of the first coils 201a and the number of the second coils 201b are the same as each other, they need not be the same as each other. Although the number of the first permanent magnets 101a and the number of the second permanent magnets 101b are the same as each other, they need not be the same as each other.

FIG. 12B and FIG. 12C are side views of FIG. 12A viewed from the Z direction. For ease of explanation, FIG. 12B illustrates the arrangement of the first coils 201a by omitting the side yoke 204a, and FIG. 12C illustrates the arrangement of the first permanent magnets 101a by further omitting the first coils 201a. Hereinafter, the first coils 201a and the first permanent magnets 101a will be described with reference to FIG. 12B and FIG. 12C. Note that the second coils 201b have the same configuration as the first coils 201a, and the second permanent magnets 101b have the same configuration as the first permanent magnets 101a.

As illustrated in FIG. 12C, the plurality of first permanent magnets 101a are attached and installed so that the plurality of first permanent magnets 101a are arranged circumferentially on one circumferential end face of the rotor 110 in the Z direction. On the other hand, as illustrated in FIG. 12B, the plurality of first coils 201a are installed circumferentially so that the plurality of first coils 201a face the plurality of first permanent magnets 101a arranged circumferentially. The rotor 110 has a yoke 102 installed on its circumferential end surface. As a result, the plurality of first permanent magnets 101a are attached to the circumferential end surface of the rotor 110 via the yoke 102. Thus, the yoke 102 is arranged on the back side of the first permanent magnet 101a. The yoke 102 is made of a metal having a magnetism such as iron, SUS400 series stainless steel, or the like to increase the magnetic force of the first permanent magnets 101a. The yoke 102 may be installed separately from or integrated with the base member of the rotor 110. The yoke 102 need not be installed in the rotor 110 and the yoke 102 need not be installed. Even in this case, it is possible to perform the levitation control and the rotation control of the rotor 110.

Here, the rotation angle, which is an angle in the Wz direction of the rotor 110, is set to θ. The rotation angle θ is an angle from the reference Oc in the Wz direction on the side of the stator 200 to the reference Or in the Wz on the side of the rotor 110. Note that the reference Oc of the Wz on the side of the stator 200 is a straight line connecting the first coil 201a-1 and the origin Os. Note that the reference Or in the Wz direction on the side of the rotor 110 is a straight line connecting the middle of the first permanent magnet 101a-1 and the first permanent magnet 101a-24 and the origin Os.

FIG. 12D is a top view of FIG. 12A viewed from the Y direction. In FIG. 12D, the side yokes 204a and 204b are omitted to illustrate the arrangement of the first coils 201a and the second coils 201b for ease of explanation. As illustrated in FIG. 12D, in the stator 200, the plurality of first coils 201a and the plurality of second coils 201b are attached and installed in plane symmetry with the XY-plane as the plane of symmetry. The stator 200 includes an X sensor 213, a Y sensor 214, Z sensors 210a, 210b, and 210c (see FIG. 14), and a Wz sensor 211 (see FIG. 12C). The X sensor 213, the Y sensor 214, the Z sensors 210a, 210b, and 210c, and the Wz sensor 211 are configured in the same manner as in the first embodiment.

FIG. 12E is a cross-sectional view taken along the line D-D' illustrated in FIG. 12B and FIG. 12C. The structure and attached position of the first coils 201a and the second coils 201b will now be described with reference to FIG. 12E. As in the first embodiment, the first coils 201a and the second coils 201b are each formed by winding a wire around a magnetic material such as a silicon steel sheet or the like used in general motors.

Specifically, as illustrated in FIG. 12E, the first coil 201a-5 is formed by winding a winding 203a-5 around a core 202a-5 formed of a magnetic material such as a silicon steel sheet and attaching a U-shaped side yoke 204a-5 to the core 202a-5. The side yoke 204a-5, like the core 202a-5, is formed of a magnetic material such as a silicon steel sheet. The core 202a-5 is arranged so that its central axis is along the Z direction. The winding 203a-5 is wound around the core 202a-5 with the central axis of the core 202a-5 as the center. The side yoke 204-5 is composed of three plate-like parts covering the outer side in the Z direction and both sides in the R-axis direction of the core 202a-5. All of the other first coils 201a have the same configuration as the first coil 201a-5.

Note that, although the configuration with the side yoke 204a attached is described as an example in the present embodiment, it is not limited to this configuration. The side yoke 204a only needs to be installed when a torque in the R-axis direction is required, and it is possible to perform levitation control and rotation control of the rotor 110 even when the side yoke 204a is not installed. By configuring the first coil 201a to include the core 202a and the side yoke 204a, torque generated in each direction can be increased as in the first embodiment.

Note that the second coil 201b has the same configuration as the first coil 201a. The core 202b, the winding 203b, and the side yoke 204b, which are components of the second coil 201b, correspond to the core 202a, the winding 203a, and the side yoke 204a, respectively, which are components of the first coils 201a.

The first permanent magnet 101a and the second permanent magnet 101b are preferably arranged in plane symmetry with the XY-plane as the plane of symmetry in the rotor 110. Further, the weight of the rotor 110 is preferably uniform in the Z direction. By making the weight of the rotor 110 uniform, the inclination and eccentricity of the rotor 110 caused by the variation of the weight can be reduced, and as a result, the control of the rotor 110 can be easily performed.

Also, the first coils 201a and the second coils 201b are preferably arranged in plane symmetry with the XY-plane as the plane of symmetry. Thus, by arranging the set of the first permanent magnets 101a and the first coils 201a and the set of the second permanent magnets 101b and the second coils 201b symmetrically, the positions where the attractive forces are generated in each set are in plane symmetry with the XY-plane as the plane of symmetry. Thus, by arranging the positions where the attractive forces are generated symmetrically, the inclination and eccentricity of the rotor 110 caused by variations in the positions can be reduced, and the control of the rotor 110 can be easily performed.

Note that, although the first permanent magnets 101a and the second permanent magnets 101b, and the first coils 201a and the second coils 201b are arranged symmetrically in the above description, it is not necessary that they are arranged symmetrically. The inclination and eccentricity of the rotor 110 can be corrected by adjusting the gap between the coils and the permanent magnets and the amount of current flowing through the coils.

Here, using the first permanent magnets 101a and the first coils 201a as examples, the arrangement of these magnets and the thrust caused by them will be described with reference to FIG. 13A to FIG. 13D. Note that the arrangement and thrust of the second permanent magnets 101b and the second coils 201b are the same as those of the first permanent magnets 101a and the first coils 201a.

FIG. 13A is a transparent view of the arrangement of the plurality of first permanent magnets 101a viewed from the Z direction. In FIG. 13A, for convenience, the plurality of first permanent magnets 101a aligned in the Wz direction are illustrated in a linear arrangement. In the present embodiment, as illustrated in FIG. 12C, twenty-four of first permanent magnets 101a are arranged. As illustrated in FIG. 13A, the twenty-four first permanent magnets 101a are arranged such that the first permanent magnets 101a-1 to the first permanent magnets 101a-24 are arranged at equal intervals in order to round the outer peripheral side surface of the rotor 110 in the Wz direction from the reference Or.

The first permanent magnets 101a are magnetized in the surface direction facing the first coils 201a. For example, the first permanent magnets 101a-1, 101a-19, and 101a-21 are magnetized to have an N-pole in the surface facing the first coil 201a, and the first permanent magnets 101a-18, 101a-22, and 101a-24 are magnetized to have an S-pole in the surface facing the first coil 201a. Further, for example, the first permanent magnets 101a-2, 101a-20, and 101a-23 are divided into two portions in the R-axis direction, and are magnetized to have an N-pole and an S-pole in the respective portions.

Thus, the plurality of first permanent magnets 101a are arranged so that, on the side facing the first coils 201a, the magnetization units, in which the N-pole, the two-division magnetization, the N-pole, the S-pole, the two-division magnetization, and the S-pole are sequentially aligned in the Wz direction, are periodically aligned in the Wz direction.

Note that the plurality of first permanent magnets 101a may include a first permanent magnet line with different magnetic poles aligned alternately in the Wz direction, which is the rotation direction, and a second permanent magnet line with different magnetic poles aligned alternately in the R-axis direction, which is a direction intersecting the Wz. The R-axis direction is a radial direction along the XY-plane with the Z-axis, which is the rotation, as the central axis. In the case illustrated in FIG. 13A, the first permanent magnets 101a-1, 101a-18, 101a-19, 101a-21, 101a-22, and 101a-24 constitute the first permanent magnet line. In the case illustrated in FIG. 13A, each of the first permanent magnets 101a-2, 101a-20, and 101a-23 constitutes the second permanent magnet line. The second permanent magnet line is not limited to the permanent magnet of two-division magnetization as illustrated in FIG. 13A, but may be composed of a plurality of permanent magnets. In this case, the plurality of permanent magnets constituting the second permanent magnet line are arranged so that different magnetic poles are arranged in the R-axis direction, which is a direction intersecting Wz. In the second permanent magnet line, different magnetic poles may be arranged not only in the R-axis direction but also in any direction intersecting with Wz.

FIG. 13B is a transparent view of the arrangement of the first coils 201a viewed from the Z direction. In the present embodiment, as illustrated in FIG. 12B, thirty-six of the first coils 201a are arranged. As illustrated in FIG. 13B, the thirty-six first coils 201a are arranged so that the first coils 201a-1 to the first coils 201a-36 are sequentially aligned at equal intervals from the reference Oc in the Wz direction in an arc shape.

FIG. 13C and FIG. 13D are diagrams schematically illustrating the magnitudes of torques in a q-axis direction, a d-axis direction and the R-axis direction generated per unit current in the first coil 201a when the rotation angle θ of the rotor 110 is at θ1, that is, thrust constants Eq, Ed and Er. The q-axis and the d-axis indicated here correspond to a q-axis and a d-axis in the motor control theory, respectively. FIG. 12B illustrates the directions of the q-axis and the d-axis with respect to the first coil 201a-10 as representative. The q-axis direction corresponds to the Wz direction, which is the circumferential direction, and the d-axis direction is the Z direction, which is the direction of the rotation axis of the rotor 110. FIG. 13C illustrates the thrust constant Eq in the q-axis direction and the thrust constant Ed in the d-axis direction. FIG. 13D illustrates the thrust constant Er in the R-axis direction.

As illustrated in FIG. 13C and FIG. 13D, the magnitude of each of the thrust constants Eq, Ed and Er depends on the rotation angle θ1 of the rotor 110 and the index j of the first coil 201a. Here, the index j is a positive integer, and an integer satisfying $1 \le j \le 36$, for example. The first coil 201a can be identified by using the index j to denote "first coil 201a-j". The thrust constants Eq, Ed, and Er can be expressed as Eq(j, 0), Ed(j, 0), and Er(j, 0), respectively, by displaying two arguments in parentheses, namely, the index j of the first coil 201a as the first argument and the rotation angle θ of the rotor 110 as the second argument. FIG. 13C and FIG. 13D illustrate the respective thrust constants when θ = θ1.

In the case illustrated in FIG. 13A and FIG. 13B where θ = θ1, the first coil 201a-10 is positioned substantially in the middle between the first permanent magnet 101a-24, whose facing face is an S-pole, and the first permanent magnet 101a-1, whose facing face is an N-pole. In this case, for example, a unit current is applied to the first coil 201a-10 so that an N-pole appears on the side facing the first permanent magnet 101a-24 and the first permanent magnet 101a-1. Note that the current is applied to the first coil 201a by the current control system 300. When the unit current is applied to the first coil 201a-10 in this manner, an attractive force acts between the first coil 201a-10 and the first permanent magnet 101a-24, and a repulsive force acts between the first coil 201a-10 and the first permanent magnet 101a-1. That is, a thrust Eq(10, θ1) is applied to the first coil 201a-10 in the q-axis direction. On the other hand, a thrust Ed(10, θ1) applied to the first coil 201a-10 in the d-axis direction is relatively small.

Further, in the case illustrated in FIG. 13A and FIG. 13B, the first coil 201a-9 faces the first permanent magnet 101a-24, whose facing surface is an S pole. In this case, for example, a unit current is applied to the first coil 201a-9 so that an S-pole appears on the side facing the first permanent magnet 101a-24. When the unit current is applied to the first coil 201a-9 in this manner, a repulsive force is applied between the first coil 201a-9 and the first permanent magnet 101a-24. That is, a thrust Ed(9, θ1) is applied to the first coil 201a-9 in the d-axis direction. On the other hand, a thrust Eq(9, θ1) applied to the first coil 201a-9 in the q-axis direction is relatively small.

Further, in the case illustrated in FIG. 13A and FIG. 13B, a unit current is applied to the first coil 201a-3 so that an N-pole appears on the side facing the first permanent magnet 101a-20. Then, since the first permanent magnet 101a-20 facing the first coil 201a-3 is magnetized by dividing it in the R-axis direction into the N-pole and the S-pole, a thrust Er(3, θ1) in the Z direction acts on the first coil 201a-3.

The force acting on the first coil 201a can be treated as equivalent to the reaction force acting on the rotor 110. Therefore, for example, when thrust is generated in the positive direction in the q-axis direction in the first coil 201a, thrust is generated in the negative direction in the q-axis direction in the rotor 110. Thus, the force acting on the rotor 110 can be controlled by the first coil 201a and the first permanent magnet 101a. In accordance with the rotation angle θ1, the forces in the q-axis direction, the d-axis direction and the R-axis direction can be controlled by individually controlling the current applied to the coils using the respective thrust constants in the direction of control.

FIG. 14 is a side view of FIG. 12A viewed from the opposite side of FIG. 12B in the Z direction. In the present embodiment, the Z sensors 210a, 210b, and 210c are installed in the same manner as in the first embodiment. In the present embodiment, as in the first embodiment, the plane ABC can be formed as illustrated in FIG. 3B based on the detected values of the Z sensors 210a, 210b, and 210c, and the displacement of the rotor 110 can be calculated using the plane ABC.

The rotation drive apparatus 10 according to the present embodiment also includes the current control system 300 illustrated in FIG. 4 as in the first embodiment, and can control the current applied to the first coils 201a and the second coils 201b. Also in the present embodiment, the attitude of the rotor 110 can be controlled according to the attitude control method described with reference to FIG. 5 in the first embodiment.

Also in the present embodiment, as in the first embodiment, the torque vector Tq indicating the torque T applied to the rotor 110 can be expressed by Expression (1). In the rotation drive apparatus 10 according to the present embodiment, as in the first embodiment, by controlling each component of the torque vector Tq by the current control system 300, the rotor 110 can be rotated while controlling the attitude of the rotor 110.

FIG. 15 is a diagram for explaining torques generated in the q-axis direction, the d-axis direction and the R-axis direction at an arbitrary position of the rotor 110. Here, FIG. 15 is used to explain that the torque vector Tq can be applied to the rotor 110 at an arbitrary rotation angle in the Wz direction of the rotor 110 by moving a magnetic field generated in the first coils 201a and the second coils 201b in accordance with the rotation of the rotor 110.

In the present embodiment, since the first coils 201a and the second coils 201b are arranged in the stator 200, torques can be generated in the q-axis direction, the d-axis direction and the R-axis direction. Each of the plurality of first permanent magnets 101a and the plurality of second permanent magnets 101b constitute a permanent magnet line including permanent magnets divided in the R-axis direction and permanent magnets not divided in the R-axis direction, respectively. In the permanent magnet line, the permanent magnets not divided in the R-axis direction mainly contribute to the torque in the q-axis direction and the d-axis direction, and the permanent magnets divided in the R-axis direction mainly contribute to the torque in the R-axis direction.

Here, the symbols used for the explanation are summarized. In FIG. 15, the symbols are shown as appropriate.

  j: Index for distinguishing the coils in the first coils 201a or the second coils 201b (j = 1 to 36)
  Ij: current value applied to the j-th first coil 201a-j or the j-th second coil 201b-j
  Φj: angle in the Wz direction of the j-th first coil 201a-j or the j-th second coil 201b-j
  r: radius of the first permanent magnet 101a or the second permanent magnet 101b
  *: multiplication symbol
  Σ: sum when the index j is changed from 1 to 36

Symbols for forces acting on the first coil 201a are as follows. Eqj, Edj and Erj shown in FIG. 15 correspond to Eqa(j, 0), Eda(j, θ) and Era(j, θ) shown below, respectively.

  Eqa(j, θ): force per unit current in the q-axis direction acting between the j-th first coil 201a-j and the rotor 110 at the rotation angle θ.
  Eda(j, θ): force per unit current in the d-axis direction acting between the j-th first coil 201a-j and the rotor 110 at the rotation angle θ.
  Era(j, θ): force per unit current in the R-axis direction acting between the j-th first coil 201a-j and the rotor 110 at the rotation angle θ.

Symbols for forces acting on the second coil 201b are as follows. Eqj, Edj and Erj shown in FIG. 15 correspond to Eqb(j, 0), Edb(j, θ) and Erb(j, θ) shown below, respectively.

Eqb(j, θ): force per unit current in the q-axis direction acting between the j-th second coil 201b-j and the rotor 110 at the rotation angle θ.
Edb(j, θ): force per unit current in the d-axis direction acting between the j-th second coil 201b-j and the rotor 110 at the rotation angle θ.
Erb(j, θ): force per unit current in the R-axis direction acting between the j-th second coil 201b-j and the rotor 110 at the rotation angle θ.

The torque vector Tq generated in the rotor 110 is represented by Expression (1) shown above. The components of the torque vector generated by the first coils 201a are represented by Txa, Tya, Tza, and Twza, and the components of the torque vector generated by the second coils 201b are represented by Txb, Tyb, Tzb, and Twzb. These components are components in the following directions.

Txa: component in the X direction of the torque vector generated by the first coils 201a
Tya: component in the Y direction of the torque vector generated by the first coils 201a
Tza: component in the Z direction of the torque vector generated by the first coils 201a
Twza: component in the Wz direction of the torque vector generated by the first coils 201a
Txb: component in the X direction of the torque vector generated by the second coils 201b
Tyb: component in the Y direction of the torque vector generated by the second coils 201b
Tzb: component in the Z direction of the torque vector generated by the second coils 201b
Twzb: component in the Wz direction of the torque vector generated by the second coils 201b

The components Txa, Tya, Tza, and Twza are represented by the following Expressions (5-1) to (5-4), respectively.

$$\mathrm{Txa} = \Sigma\{\mathrm{Era}(j, \theta) * \cos\Phi j * \mathrm{Ij}\} \qquad \text{... Expression (5-1)}$$

$$\mathrm{Tya} = \Sigma\{\mathrm{Era}(j, \theta) * \sin\Phi j * \mathrm{Ij}\} \qquad \text{... Expression (5-2)}$$

$$\mathrm{Tza} = \Sigma\{\mathrm{Eda}(j, \theta) * \mathrm{Ij}\} \qquad \text{... Expression (5-3)}$$

$$\mathrm{Twza} = \Sigma\{\mathrm{Eqa}(j, \theta) * \mathrm{Ij} * r\} \qquad \text{... Expression (5-4)}$$

The components Txb, Tyb, Tzb, and Twzb are represented by the following Expressions (5-5) to (5-8), respectively.

$$\mathrm{Txb} = \Sigma\{\mathrm{Erb}(j, \theta) * \cos\Phi j * \mathrm{Ij}\} \qquad \text{... Expression (5-5)}$$

$$\mathrm{Tyb} = \Sigma\{\mathrm{Erb}(j, \theta) * \sin\Phi j * \mathrm{Ij}\} \qquad \text{... Expression (5-6)}$$

$$\mathrm{Tzb} = \Sigma\{\mathrm{Edb}(j, \theta) * \mathrm{Ij}\} \qquad \text{... Expression (5-7)}$$

$$\mathrm{Twzb} = \Sigma\{\mathrm{Eqb}(j, \theta) * \mathrm{Ij} * r\} \qquad \text{... Expression (5-8)}$$

Here, as illustrated in FIG. 12D, it is assumed that the first coils 201a and the second coils 201b are arranged at a distance t from the XY-plane in the Z direction in plane symmetry with the XY-plane as the plane of symmetry. Then, the components Tx, Ty, Tz, Twx, Twy, and Twz of the torque vector Tq are represented by the following Expressions (6-1) to (6-6), respectively.

$$Tx = Txa + Txb \qquad \text{... Expression (6-1)}$$

$$Ty = Tya + Tyb \qquad \text{... Expression (6-2)}$$

$$Tz = Tza - Tzb \qquad \text{... Expression (6-3)}$$

$$Twx = (Tya - Tyb) * t \qquad \text{... Expression (6-4)}$$

$$Twy = (Txa - Txb) * t \qquad \text{... Expression (6-5)}$$

$$Twz = Twza + Twzb \qquad \text{... Expression (6-6)}$$

To apply the desired torque vector Tq, the current Ij satisfying the above Expressions (5-1) to (5-8) and (6-1) to (6-6) may be applied to each of the first coils 201a and the second coils 201b. The current control system 300 can control the application of the current Ij as such. Note that each coil generates some torque in the d-axis direction. The torque in the d-axis direction generated by each coil can be cancelled out by arranging the first coils 201a and the second coils 201b in plane symmetry with the XY-plane as the plane of symmetry, because the directions of the torques are opposite to each other.

Further torque may be applied to the rotor 110 by the operation of controlling the current application to the first coils 201a and the second coils 201b. For example, when the rotor 110 starts to levitate, the rotor 110 must be moved to a levitation position. In this case, the torque in the Y direction may be insufficient. When the rotor 110 is moved to the levitation position, the torque for rotating the rotor 110 is unnecessary, so that the torque for rotation can be diverted to the torque for levitation. Specifically, a torque Tya_q in the Y direction represented by the following Expression (7-1) is obtained as the torque for levitation by flowing currents through the first coils 201a at positions each facing the first permanent magnet 101a which is not divided in the R-axis direction among the plurality of first permanent magnets 101a. Similarly, a torque Tyb_q in the Y direction represented by the following Expression (7-2) is obtained as the torque for levitation by flowing currents through the second coils 201b at positions each facing the second permanent magnet 101b which is not divided in the R-axis direction among the plurality of second permanent magnets 101b.

$$Tya\_q = \Sigma\{Eqa(j, \theta) * \cos\Phi j * Ij\} \qquad \text{... Expression (7-1)}$$

$$Tyb\_q = \Sigma\{Eqb(j, \theta) * \cos\Phi j * Ij\} \qquad \text{... Expression (7-2)}$$

Further adding Tya_q and Tyb_q represented by Expressions (7-1) and (7-2) to Ty represented by Expression (6-2) yields a torque in the Y direction represented by the following Expression (7-3). The torque Ty in the Y direction represented by the following Expression (7-3) can be applied to the rotor 110, for example, when the rotor 110 is moved to the levitation position.

$$Ty = Tya + Tyb + Tya\_q + Tyb\_q \qquad \text{... Expression (7-3)}$$

Thus, the rotation drive apparatus 10 according to the present embodiment can control the rotation speed and the attitude of the rotor 110 because the current control system 300 can apply the torque vector Tq having the components of six axes to the rotor 110 as described above.

FIG. 16 is a schematic diagram illustrating the attractive force acting between the first permanent magnet 101a of the rotor 110 and the core 202a of the first coil 201a. The attractive force acting between the core 202a of the first coil 201a and the first permanent magnet 101a will be described with reference to FIG. 16. Note that the second coil 201b and the second permanent magnet 101b have the same structure, and the same attractive force acts between them.

The core 202a arranged within the first coil 201a is made of a magnetic material. Therefore, as illustrated in FIG. 16, an attraction force fma, which is a magnetic force, is generated between the core 202a and the first permanent magnet 101a. The attraction force fma is balanced by the gravity of the rotor 110 at a position where the first permanent magnet 101a is slightly lower than the core 202a in the Y direction due to the gravity of the rotor 110. The attraction force fm is decomposed into an attraction force fmaz in the Z direction and an attraction force fmay in the Y direction. The combined force of the attraction forces fmay in the Y direction applied to the first coils 201a-1 to 201a-36 is an attraction force Fmay generated in the gravity direction of the rotor 110. Similarly, the combined force of the attraction forces fmby in the Y direction applied to the second coils 201b-1 to 201b-36 is an attraction force Fmby generated in the gravity direction of the rotor 110. In the present embodiment, the position where the attractive force Fmay + Fmby in the Y direction integrated for the first coils 201a and the second coils 201b is equal to the gravity of the rotor 110 is the levitation position of the rotor 110.

Also in the present embodiment, as described above, the attractive force Fmay in the Y direction acts on the rotor 110 because the first coils 201a have the cores 202a. Also, the attractive force Fmby in the Y direction acts on the rotor 110 similarly to the attractive force Fmay because the second coils 201b have the cores 202b. The attractive forces Fmay and Fmby in the Y direction are used as a force for levitating the rotor 110 in the Y direction. The first coil 201a and the second coil 201b are not necessarily limited to a cored coil having a core, and may be an air-core coil. In the case of the air-core coil, the torque Ty in the Y direction is used as the force for levitating the rotor 110 in the Y direction.

On the other hand, the combined force obtained by adding the attractive forces fmaz in the Z direction acting on the first coils 201a-1 to 201a-36 is an attractive force Fmaz generated in the Z direction of the rotor 110. Similarly, the combined force obtained by adding the attractive forces fmbz in the Z direction acting on the second coils 201b-1 to 201b-36 is an attractive force Fmbz generated in the Z direction of the rotor 110. In the Z direction, the first coils 201a and the second coils 201b are arranged in plane symmetry with the XY-plane as the plane of symmetry. Therefore, both the attractive force Fmaz and the attractive force Fmbz are generated in plane symmetry with the XY-plane of symmetry. By arranging the first coils 201a and the second coils 201b in plane symmetry with the XY-plane of symmetry, the attractive forces Fmaz and Fmbz in the Z direction generated by the first coils 201a and the second coils 201b, respectively, have the same magnitude in opposite directions and cancel out each other.

As described above, according to the present embodiment, the levitation control and the rotation control of the rotor 110 can be performed with a simple configuration without requiring numerous actuators. Therefore, according to the present embodiment, the rotor 110 can be levitated to perform the rotation control of the rotor 110 while avoiding enlargement of the whole rotation drive apparatus 10.

Note that the configuration of the present embodiment can be combined with any of the configurations of the first to third embodiments. That is, the first permanent magnets 101a, the second permanent magnets 101b, the first coils 201a, and the second coils 201b may also be installed in the rotation drive apparatus 10 according to the present embodiment as in any of the first to third embodiments. In the rotation drive apparatus 10 according to the present embodiment, the first coils 201a and the second coils 201b may also be installed inside the rotor 110 as in the combination of the second and third embodiments.

[Fifth Embodiment]

A rotation drive apparatus according to a fifth embodiment of the present invention will be described with reference to FIG. 17A to FIG. 18C. Note that the same components as those in the above first to fourth embodiments are labeled with the same references, and the description thereof will be omitted or simplified.

First, the configuration of the rotation drive apparatus according to the present embodiment will be described with reference to FIG. 17A to FIG. 17D. FIG. 17A is a perspective view illustrating the rotation drive apparatus 10 according to the present embodiment. FIG. 17B is a side view of the rotation drive apparatus 10 according to the present embodiment viewed in the Z direction. FIG. 17C is a top view of the rotation drive apparatus 10 according to the present embodiment viewed in the Y direction. FIG. 17D is a cross-sectional view taken along a line E-E' illustrated in FIG. 17B. For ease of explanation, the side yokes 204a are omitted in FIG. 17B and auxiliary yoke 402 described later is omitted in FIG. 17C.

As illustrated in FIG. 17A to FIG. 17D, in the rotation drive apparatus 10 according to the present embodiment, auxiliary permanent magnets 401a and 401b and an auxiliary yoke 402 are installed in the stator 200. The configuration of the rotation drive apparatus 10 according to the present embodiment is the same as that of the fourth embodiment except that the auxiliary permanent magnets 401a and 401b and the auxiliary yoke 402 are installed.

A plurality of the auxiliary permanent magnets 401a and 401b are arranged along the circumferential direction along the Wz direction so as to face the outer peripheral side surface of the rotor 110 above the rotor 110 in the Y direction. The plurality of auxiliary permanent magnets 401a and 401b are magnetized in the R axis direction, for example. The plurality of auxiliary permanent magnets 401a and 401b are arranged in plane symmetry of the XY-plane and the YZ-plane. Note that the plurality of auxiliary permanent magnets may not be arranged, and a single auxiliary permanent magnet may be arranged.

For example, two auxiliary permanent magnets 401a-1 and 401a-2 are arranged as the auxiliary permanent magnets 401a on the side of the first coil 201a in the Z direction. Two auxiliary permanent magnets 401b-1 and 401b-2 are arranged as the auxiliary permanent magnets 401b on the side of the second coil 201b in the Z direction. The auxiliary permanent magnets 401a-1 and 401a-2 and the auxiliary permanent magnets 401b-1 and 401b-2 are arranged in plane symmetry with the XY-plane as the plane of symmetry. Further, the auxiliary permanent magnets 401a-1 and 401b-1 and the auxiliary permanent magnets 401a-2 and 401b-2 are arranged in plane symmetry with the YZ-plane as the plane of symmetry. Note that the numbers and the arrangements of the auxiliary permanent magnets 401a and 401b are not particularly limited and can be appropriately changed. The auxiliary permanent magnets 401a and 401b may be one permanent magnet in total or one or more permanent magnets, respectively.

The auxiliary yoke 402 is arranged on the back surface of the auxiliary permanent magnets 401a and 401b. The auxiliary yoke 402 is one yoke arranged in common with the plurality of auxiliary permanent magnets 401a and 401b. A separate auxiliary yoke 402 may be arranged in each for the plurality of auxiliary permanent magnets 401a and 401b.

The rotor 110 is made of a magnetic material such as iron. Thus, an attractive force is generated between the rotor 110 and the auxiliary permanent magnets 401a and 401b. Note that, although the material of the rotor 110 does not necessarily have to be magnetic, in this case, a magnetic member such as an iron plate can be installed on the outer peripheral side of the rotor 110, which faces the auxiliary permanent magnets 401a and 401b to achieve the same effect.

FIG. 18A, FIG. 18B, and FIG. 18C are schematic diagrams illustrating the rotor 110 moved in the Y direction while the center position of the rotor 110 in the X direction is aligned with the origin Os. The levitation of the rotor 110 will be described with reference to FIG. 18A to FIG. 18C.

The rotor 110 is arranged at a position where its center O overlaps with the origin Os. As illustrated in FIG. 18A to FIG. 18C, in the present embodiment, the auxiliary permanent magnets 401a and 401b are arranged near the rotor 110. Therefore, attractive forces Fmah and Fmbh are generated between the auxiliary permanent magnets 401a and 401b and the rotor 110, respectively. When the rotor 110 is moved in the Y direction, there is an equilibrium point ep at which the magnitude of the gravity force Fg of the rotor 110 is equal to the magnitude of the attractive force Fmay + Fmby + Fmah + Fmbh. Note that, in the present embodiment, it is configured that the origin Os and the equilibrium point ep overlap each other. FIG. 18A illustrates a state in which the rotor 110 is arranged so that the center O of the rotor 110 overlaps the equilibrium point ep.

For example, if the position of the rotor 110 is shifted in the positive direction in the Y direction as illustrated in FIG. 18B from the state illustrated in FIG. 18A, the attraction force Fmay + Fmby + Fmah + Fmbh becomes larger than the gravity force Fg. On the other hand, if the position is shifted in the negative direction in the Y direction as illustrated in FIG. 18C from the state illustrated in FIG. 18A, the gravity force Fg becomes larger than the attractive force Fmay + Fmby + Fmah + Fmbh. The rotor 110 moves in the direction of the larger force, i.e., the attractive force Fmay + Fmby + Fmah + Fmbh in the case illustrated in FIG. 18B, and the gravity force Fg in the case illustrated in FIG. 18C.

Accordingly, the rotation drive apparatus 10 applies a current to the first coils 201a and the second coils 201b by the current control system 300 at a position where the center O of the rotor 110 overlaps with the equilibrium point ep, and controls the torque in the Y direction applied to the rotor 110. Further, the rotation drive apparatus 10 can maintain the levitation of the rotor 110 by similarly controlling the torque applied to the rotor 110 in the X direction.

In order to maintain the levitation of the rotor 110, it is desirable to control the torque applied to the rotor 110 at a position where the center O of the rotor 110 is overlapped with the equilibrium point ep. Thus, the levitation state of the rotor 110 can be continued at a smaller current than the current flowing through the first coil 201a and the second coil 201b. Further, as described above, the currents flowing through the first coils 201a and the second coils 201b so as to generate torque in the Wz direction can rotate the rotor 110 in the Wz direction while the rotor 110 is levitated so that the center O of the rotor 110 overlaps the equilibrium point ep. Thus, the rotation drive apparatus 10 can rotate the rotor 110 in the Wz direction while levitating in the Y direction by controlling the current flowing through the first coils 201a and the second coils 201b by the current control system 300 to control the coils with respect to the rotor 110.

Note that, in the present embodiment, the rotor 110 can be arranged at a position where the center O of the rotor 110 overlaps the equilibrium point ep in the same manner as in the first embodiment.

The configuration of the present embodiment including the auxiliary permanent magnets 401 is particularly effective when the weight of the rotor 110 is large. In the present embodiment, while the gravity of the rotor 110 is supported by the attraction force of the auxiliary permanent magnets 401, the levitation of the rotor 110 can be controlled at the equilibrium point ep by the torque of the first coils 201a and the second coils 201b. Thus, in the present embodiment, the control of the rotor 110 can be performed with small power.

Note that the magnetization directions of the auxiliary permanent magnets 401a and 401b are not particularly limited, and no matter which directions the magnetization directions are, attractive forces are generated between the auxiliary permanent magnets 401a and 401b and the rotor 110. For example, the auxiliary permanent magnets 401a and 401b are magnetized in the R-axis direction so that their surfaces facing the rotor 110 become N-poles or S-poles.

In particular, when the auxiliary permanent magnets 401a and 401b are magnetized in the R-axis direction, it is desirable that the auxiliary permanent magnets 401a and 401b are arranged in the direction in which the magnetic fluxes of the auxiliary permanent magnets 401a and 401b converges. In this case, the influence of the magnetic fluxes of the auxiliary permanent magnets 401a and 401b on the surroundings can be reduced. Specifically, for example, it is assumed that the auxiliary permanent magnets 401a-1 are magnetized so that the facing surface on the side of the rotor 110 is an N-pole, and the auxiliary permanent magnets 401a-2 are magnetized so that the facing surface on the side of the rotor 110 is an S-pole. Similarly, it is assumed that the auxiliary permanent magnets 401b-1 are magnetized so that the facing surface on the side of the rotor 110 is an S-pole, and the auxiliary permanent magnets 401b-2 are magnetized so that the facing surface on the side of the rotor 110 is an N-pole. It is desirable that the plurality of auxiliary permanent magnets 401a-1, 401a-2, 401b-1, and 401b-2 are arranged so that the magnetic poles adjacent to each other on the side of the rotor 110 are different in this manner. In this arrangement, the magnetic fluxes exiting from the N-pole enters the adjacent S-pole, thereby reducing the leakage magnetic fluxes to the periphery. Thus, the influence of the magnetic fluxes of the auxiliary permanent magnets 401a and 401b on the periphery is reduced.

Note that, although the present embodiment has been described as an example in which the auxiliary permanent magnets 401a and 401b are installed as the auxiliary magnets, the electromagnets may be arranged as the auxiliary magnets instead of the auxiliary permanent magnets 401a and 401b. When the electromagnets are installed, the strength of the attraction force can be controlled by changing the current flowing through the electromagnets according to the state of the rotor 110, and further stable levitation control of the rotor 110 can be performed. In the case of the electromagnets, it is desirable that they are magnetized in the same direction as the auxiliary permanent magnets 401a and 401b.

[Sixth Embodiment]

A rotating apparatus according to a sixth embodiment of the present invention will be described with reference to FIG. 19A and FIG. 19B. Note that the same components as those in the first to fifth embodiments are labeled with the same references, and the description thereof will be omitted or simplified.

The rotation drive apparatus 10 according to the above-described embodiments can be used for rotation equipment utilizing the rotation of the rotor 110. In the present embodiment, an X-ray computed tomography (CT) apparatus will be described as an example of the rotation equipment using the rotation drive apparatus 10 according to the above-described embodiments.

FIG. 19A is a side view illustrating an X-ray CT apparatus 600 in which the rotation drive apparatus 10 according to the first embodiment is used. FIG. 19A corresponds to the side view of the rotation drive apparatus 10 according to the first embodiment illustrated in FIG. 1B.

As illustrated in FIG. 19A, the X-ray CT apparatus 600 includes the rotation drive apparatus 10 according to the first embodiment, a plate-like part 602, an X-ray irradiation unit 604, an X-ray detection unit 606, a signal amplification unit 608, a cooling unit 610, power supply units 612 and 614, and a power supply control unit 616. The rotation drive apparatus 10 is installed on a mounting (not illustrated). The plate-like part 602, the X-ray irradiation unit 604, the X-ray detection unit 606, the signal amplification unit 608, the cooling unit 610, the power supply units 612 and 614, and the power supply control unit 616 are components installed in the rotor 110 of the rotation drive apparatus 10, respectively.

The plate-like part 602 is a circular plate-like member installed in the cylindrical rotor 110 along the XY-plane. In the center of the plate-like part 602, an imaging port 618 for inserting a subject such as a human or other organism, or the like as an object of X-ray CT is provided. The imaging port 618 is a circular opening centered on the Z-axis which is the rotation axis of the rotor 110. The plate-like part 602 rotates in the Wz direction along with the rotation of the rotor 110 in the Wz direction with the Z-axis as the rotation axis.

Although the manner in which the subject is inserted into the inside of the imaging port 618 is not particularly limited, for example, the subject is inserted into the inside of the imaging port 618 by sliding a bed (not illustrated) on which the subject lies sideways along the Z direction in the Z direction into the inside of the imaging port 618. Note that the subject is not necessarily limited to a living organism such as a human being or the like, but may be an article such as an industrial product.

The X-ray irradiation unit 604, the X-ray detection unit 606, the signal amplification unit 608, the cooling unit 610, the power supply units 612 and 614, and the power supply control unit 616 are mounted on one surface of the plate-like part 602 in the Z direction. The X-ray irradiation unit 604, the X-ray detection unit 606, the signal amplification unit 608, the cooling unit 610, the power supply units 612 and 614, and the power supply control unit 616 rotate in the Wz direction with the Z-axis as the rotation axis by the rotation of the plate-like part 602 accompanied with the rotation of the rotor 110 by the rotation drive apparatus 10. Note that the respective units may be mounted on one or the other surfaces or both surfaces of the plate-like part 602 in the Z direction. The respective units may be arranged between the plurality of coil lines.

The X-ray irradiation unit 604 and the X-ray detection unit 606 are arranged on the same surface of the plate-like part 602 in the Z direction. The X-ray irradiation unit 604 and the X-ray detection unit 606 are arranged so as to face each other in the diameter direction of the annular plate-like part 602 across the Z-axis which is the rotation axis thereof.

The X-ray irradiation unit 604 is an irradiation unit that irradiates the subject with X-rays which are radiation while the rotor 110 rotates to make one rotation around the subject. The X-ray irradiation unit 604 is, for example, an X-ray tube. The power supply units 612 and 614 are power supply units that supply a voltage for X-ray irradiation such as a tube voltage to the X-ray tube or the like to the X-ray irradiation unit 604. The power supply control unit 616 is a control device that controls supply of the tube voltage by the power supply units 612 and 614. The cooling unit 610 is a cooling device that cools the X-ray irradiation unit 604, which generates heat during the X-ray irradiation.

The X-ray detection unit 606 is a detector that detects the X-ray irradiated from the X-ray irradiation unit 604 toward the subject and transmitted through the subject. The X-ray detection unit 606 outputs a detection signal corresponding to the detected X-ray. The signal amplification unit 608 amplifies the detection signal output from the X-ray detection unit 606. The signal amplification unit 608 outputs the amplified detection signal to a system control unit (not illustrated). The system control unit generates an X-ray CT image by image processing or the like based on the detection signal from the signal amplification unit 608.

As described above, in the X-ray CT apparatus 600, the rotation drive apparatus 10 can be used as an apparatus that rotates the X-ray irradiation unit 604, the X-ray detection unit 606, and the like by rotating the rotor 110.

The rotation drive apparatus 10 according to other embodiments other than the first embodiment can also be used in the X-ray CT apparatus in the same manner. For example, FIG. 19B is a side view illustrating an X-ray CT apparatus 600 in which the rotation drive apparatus 10 according to the fourth embodiment is used. FIG. 19B corresponds to the side view of the rotation drive apparatus 10 according to the fourth embodiment illustrated in FIG. 12B.

In the case illustrated in FIG. 19B, the X-ray CT apparatus 600 includes the rotation drive apparatus 10 according to the fourth embodiment, the plate-like part 602, the X-ray irradiation unit 604, the X-ray detection unit 606, the signal amplification unit 608, the cooling unit 610, the power supply units 612 and 614, and the power supply control unit 616. In this case also, as in the case illustrated in FIG. 19A, the plate-like part 602, the X-ray irradiation unit 604, the X-ray detection unit 606, the signal amplification unit 608, the cooling unit 610, the power supply units 612 and 614, and the power supply control unit 616 can be installed in the rotor 110.

In addition to the cases illustrated in FIG. 19A and FIG. 19B, the X-ray CT apparatus may be configured by using the rotation drive apparatus 10 according to any of the first to fifth embodiments or any combination thereof.

Note that, although the X-ray CT apparatus 600 using X-ray has been described in the present embodiment, it is possible to configure a CT apparatus using radiation such as gamma ray instead of X-ray. In this case, the irradiation unit corresponding to the type of the radiation can be used instead of the X-ray irradiation unit 604, and the detection unit corresponding to the type of the radiation can be used instead of the X-ray detection unit 606.

[Modification Embodiment]

The present invention is not limited to the above embodiments, and various modifications are possible.

For example, in the above embodiment, the X-ray CT apparatus 600 has been described as an example of the rotation equipment in which the rotation drive apparatus 10 is used, but the rotation equipment is not limited thereto. As the rotation equipment in which the rotation drive apparatus 10 is used, any apparatus other than the X-ray CT apparatus 600 can be configured. Further, according to the type of the rotation equipment, components installed in the rotor 110 and rotating together with the rotor 110 can be designed.

The present invention can also be implemented by a process in which a program for implementing one or more functions of the above-described embodiments are supplied to a system or an apparatus via a network or storage medium, and one or more processors in the computer of the system or the apparatus read and execute the program. It can also be implemented by a circuit (For example, an ASIC) for implementing the one or more functions.

The present invention is not limited to the above embodiments, and various modifications and variations can be made without departing from the spirit and scope of the present invention. Accordingly, the following claims are attached to make the scope of the present invention public.

This application is based upon and claims the benefit of priority from Japanese Patent Application No. 2022-210208, filed on December 27, 2022, the disclosure of which is incorporated herein in its entirety by reference.

[Reference Signs List]

10 rotation drive apparatus
110 rotor
101a first permanent magnet
101b second permanent magnet
102 yoke
200 stator
201a first coil
201b second coil
202a, 202b core
203a, 203b winding
204a, 204b side yoke

210a, 210b, 210c Z sensor
211 Wz sensor
212 scale
213 X sensor
214 Y sensor
300 current control system
301 motor controller
312a, 312b current sensor
313a, 313b current controller
401a, 401b auxiliary permanent magnet
402 auxiliary yoke
501 attitude controller
502 current calculator
600 X-ray CT apparatus
602 plate-like part
604 X-ray irradiation unit
606 X-ray detection unit
608 signal amplification unit
610 cooling unit
612 power supply unit
614 power supply unit
616 power supply control unit
618 imaging port

## Claims

1. A rotation drive apparatus comprising:

   a rotor that includes a plurality of magnets and is rotatable in a rotation direction with a rotation axis intersecting a direction of gravity; and
   a stator that includes a plurality of coils,
   wherein the plurality of magnets includes a magnet group arranged circumferentially along the rotation direction on an outer peripheral side surface of the rotor,
   wherein the magnet group includes a first magnet line with different magnetic poles aligned in the rotation direction and a second magnet line with different magnetic poles aligned in a direction intersecting the rotation direction, and
   wherein the plurality of coils includes, above the rotor, a coil group that is arranged in an arc shape along the rotation direction and can face the magnet group.

2. The rotation drive apparatus according to claim 1, wherein a coil of the coil group includes a core that generates an attractive force with a magnet of the magnet group.

3. The rotation drive apparatus according to claim 1 or 2, wherein the plurality of coils are arranged in plane symmetry with a plane including the rotation axis and along the direction of gravity as a plane of symmetry.

4. The rotation drive apparatus according to any one of claims 1 to 3,

   wherein the magnet group is arranged in a plurality of lines on the outer peripheral side surface, and
   wherein the coil group is arranged in a plurality of lines to face the plurality of magnets arranged in the plurality of lines.

5. The rotation drive apparatus according to claim 4,

   wherein the magnet group is arranged in two lines on the outer peripheral side surface on a side of one end and on a side of the other end of the rotor in a direction along the rotation axis, and
   wherein the coil group is arranged in two lines to face the magnet group arranged in the two lines.

6. The rotation drive apparatus according to claim 4 or 5,

   wherein the stator includes a sensor that detects displacement of the rotor, and
   wherein the sensor is installed between lines of the coil groups.

7. The rotation drive apparatus according to any one of claims 1 to 6,

   wherein the plurality of magnets further includes another magnet group arranged circumferentially along the rotation direction on an inner peripheral side surface of the rotor,
   wherein the another magnet group includes a first magnet line and a second magnet line,
   wherein the plurality of coils further includes, inside the rotor, another coil group that is arranged in an arc shape along the rotation direction and can face the plurality of magnets arranged on the inner peripheral side surface.

8. The rotation drive apparatus according to any one of claims 1 to 7, wherein the plurality of coils further includes another coil group that is arranged in an arc shape along the rotation direction in a circumference including an arc in which the coil groups are arranged and can face the magnet groups.

9. The rotation drive apparatus according to claim 8, wherein the coil group and the another coil group are arranged circumferentially along the rotation direction.

10. The rotation drive apparatus according to claim 8 or 9, wherein each of coils of the another other coil group is an air-core coil.

11. The rotation drive apparatus according to any one of claims 1 to 10, wherein, in the second magnet line, different magnetic poles are aligned in a direction along the rotation axis.

12. The rotation drive apparatus according to any one of claims 1 to 11, wherein, the magnet group is arranged so that at least one of the second magnet line faces the coil when the rotor is rotating.

13. A rotation drive apparatus comprising:

    a rotor that includes a plurality of magnets and is rotatable in a rotation direction with a rotation axis intersecting a direction of gravity; and
    a stator that includes a plurality of coils,
    wherein the plurality of magnets includes a first magnet group arranged circumferentially along the rotation direction at one end face of the rotor in a direction along the rotation axis and a second magnet group arranged circumferentially along the rotation direction at the other end face of the rotor in a direction along the rotation axis,
    wherein the first magnet group and the second magnet group each include a first magnet line with different magnetic poles aligned in the rotation direction and a second magnet line with different magnetic poles aligned in a direction intersecting the rotation direction, and
    wherein the plurality of coils includes a first coil group that is arranged circumferentially along the rotation direction and can face the first magnet group, and a second coil group that is arranged circumferentially along the rotation direction and can face the second magnet group.

14. The rotation drive apparatus according to claim 13,

    wherein a coil of the first coil group includes a core that generates an attractive force with a magnet of the first magnet group, and
    wherein a coil of the second coil group includes a core that generates an attractive force with a magnet of the second magnet group.

15. The rotation drive apparatus according to claim 13 or 14, comprising an auxiliary magnet that is arranged above the rotor and generates an attraction force with the rotor.

16. The rotation drive apparatus according to claim 15, wherein the auxiliary magnets are arranged in plane symmetry with a plane including the rotation axis and along the direction of gravity as a plane of symmetry.

17. The rotation drive apparatus according to claim 15 or 16,

wherein a plurality of the auxiliary magnets are arranged, and

wherein the plurality of auxiliary magnets are arranged so that magnetic poles adjacent to each other on a side of the rotor are different.

18. The rotation drive apparatus according to any one of claims 13 to 17, wherein the first coil group and the second coil group are arranged in plane symmetry with a plane orthogonal to the rotation axis as a plane of symmetry.

19. The rotation drive apparatus according to any one of claims 13 to 18, wherein, in the second magnet line, the different magnetic poles are aligned in a radial direction with the rotation axis as a central axis.

20. The rotation drive apparatus according to any one of claims 13 to 19, comprising a control unit that controls drive of the rotor by controlling currents applied to the plurality of coils.

21. A method of controlling a rotation drive apparatus including: a rotor that includes a plurality of magnets and is rotatable in a rotation direction with a rotation axis intersecting a direction of gravity; and a stator that includes a plurality of coils, wherein the plurality of magnets includes a magnet group arranged circumferentially along the rotation direction on an outer peripheral side surface of the rotor, wherein the magnet group includes a first magnet line with different magnetic poles aligned in the rotation direction and a second magnet line with different magnetic poles aligned in a direction intersecting the rotation direction, and wherein the plurality of coils includes, above the rotor, a coil group that is arranged in an arc shape along the rotation direction and can face the magnet group, the method comprising controlling drive of the rotor by controlling currents applied to the plurality of coils.

22. A method of controlling a rotation drive apparatus including: a rotor that includes a plurality of magnets and is rotatable in a rotation direction with a rotation axis intersecting a direction of gravity; and a stator that includes a plurality of coils, wherein the plurality of magnets includes a first magnet group arranged circumferentially along the rotation direction at one end face of the rotor in a direction along the rotation axis and a second magnet group arranged circumferentially along the rotation direction at the other end face of the rotor in a direction along the rotation axis, wherein the first magnet group and the second magnet group each include a first magnet line with different magnetic poles aligned in the rotation direction and a second magnet line with different magnetic poles aligned in a direction intersecting the rotation direction, and wherein the plurality of coils includes a first coil group that is arranged circumferentially along the rotation direction and can face the first magnet group, and a second coil group that is arranged circumferentially along the rotation direction and can face the second magnet group, the method comprising controlling drive of the rotor by controlling currents applied to the plurality of coils.

23. Rotation equipment comprising:

the rotation drive apparatus according to any one of claims 1 to 20; and
a component installed in the rotor.

24. The rotation equipment according to claim 23, wherein the component includes:

an irradiation unit that irradiates a subject with radiation, the subject being arranged inside the rotor; and
a detection unit that is arranged to face the irradiation unit across the rotation axis and detects the radiation transmitted through the subject.

# FIG. 1A

# FIG. 1B

# FIG. 1C

# FIG. 1D

EP 4 641 891 A1

# FIG. 2A

FIG. 2B

# FIG. 2C

EP 4 641 891 A1

FIG. 2D

# FIG. 3A

# FIG. 3B

# FIG. 4

# FIG. 5

EP 4 641 891 A1

FIG. 6

# FIG. 7A

202a

101a

fmay    fmar

fmax

# FIG. 7B

# FIG. 8A

# FIG. 8B

# FIG. 8C

# FIG. 9A

# FIG. 9B

# FIG. 9C

# FIG. 10A

# FIG. 10B

# FIG. 10C

# FIG. 10D

# FIG. 11A

# FIG. 11B

# FIG. 12A

EP 4 641 891 A1

# FIG. 12B

# FIG. 12C

# FIG. 12D

EP 4 641 891 A1

# FIG. 12E

# FIG. 13A

EP 4 641 891 A1

FIG. 13B

EP 4 641 891 A1

# FIG. 13C

EP 4 641 891 A1

EZ(3,θ1)

# FIG. 14

# FIG. 15

# FIG. 16

202a

101a

102

fma

fmay

fmaz

# FIG. 17A

# FIG. 17B

# FIG. 17C

# FIG. 17D

EP 4 641 891 A1

# FIG. 18A

# FIG. 18B

# FIG. 18C

# FIG. 19A

# FIG. 19B

# INTERNATIONAL SEARCH REPORT

| International application No. |
| --- |
| **PCT/JP2023/045875** |

## A. CLASSIFICATION OF SUBJECT MATTER

*H02K 7/09*(2006.01)i; *A61B 6/03*(2006.01)i; *F16C 32/04*(2006.01)i; *H02K 21/16*(2006.01)i
FI:    H02K7/09; A61B6/03 521D; F16C32/04 A; H02K21/16 M

According to International Patent Classification (IPC) or to both national classification and IPC

## B. FIELDS SEARCHED

Minimum documentation searched (classification system followed by classification symbols)

H02K7/09; A61B6/03; F16C32/04; H02K21/16

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

## C. DOCUMENTS CONSIDERED TO BE RELEVANT

| Category* | Citation of document, with indication, where appropriate, of the relevant passages | Relevant to claim No. |
| --- | --- | --- |
| X | JP 2021-126001 A (CANON KABUSHIKI KAISHA) 30 August 2021 (2021-08-30) paragraphs [0011]-[0043], fig. 1-2 | 1-3, 8-9, 11, 20, 21, 23 |
| Y | | 4-14, 18-20, 22-24 |
| A | | 15-17 |
| Y | JP 2001-190045 A (SANKYO SEIKI MFG CO., LTD.) 10 July 2001 (2001-07-10) paragraphs [0033]-[0035], fig. 5 | 4-12, 20, 23, 24 |
| Y | JP 2015-173584 A (KITADA, Yasuo) 01 October 2015 (2015-10-01) paragraphs [0033]-[0034], fig. 12-13 | 7-12, 20, 23, 24 |
| Y | JP 2014-33543 A (IBARAKI UNIVERSITY) 20 February 2014 (2014-02-20) paragraphs [0020]-[0028], fig. 1 | 13-14, 18-20, 22-24 |
| Y | JP 2014-180578 A (AKTIEBOLAGET SKF) 29 September 2014 (2014-09-29) paragraphs [0113]-[0114], fig. 1 | 24 |

☐ Further documents are listed in the continuation of Box C.    ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **27 February 2024** | **12 March 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

International application No.

**PCT/JP2023/045875**

| Patent document cited in search report | | | Publication date (day/month/year) | Patent family member(s) | | | Publication date (day/month/year) |
|---|---|---|---|---|---|---|---|
| JP | 2021-126001 | A | 30 August 2021 | US | 2022/0376596 | A1 | |
| | | | | paragraphs [0029]-[0061], fig. 1A-2D | | | |
| | | | | WO | 2021/157359 | A1 | |
| JP | 2001-190045 | A | 10 July 2001 | US | 2001/0013734 | A1 | |
| | | | | paragraphs [0131]-[0139], fig. 10(a) | | | |
| | | | | EP | 1115193 | A2 | |
| | | | | KR | 10-2001-0070351 | A | |
| JP | 2015-173584 | A | 01 October 2015 | US | 2017/0098989 | A1 | |
| | | | | paragraphs [0095]-[0096], fig. 12-13 | | | |
| | | | | WO | 2016/103740 | A1 | |
| | | | | EP | 3240163 | A1 | |
| | | | | CN | 106416023 | A | |
| JP | 2014-33543 | A | 20 February 2014 | (Family: none) | | | |
| JP | 2014-180578 | A | 29 September 2014 | US | 2014/0270051 | A1 | |
| | | | | paragraphs [0165]-[0166], fig. 1 | | | |
| | | | | DE | 102014204767 | A1 | |
| | | | | CN | 104545965 | A | |

Form PCT/ISA/210 (patent family annex) (July 2022)

**REFERENCES CITED IN THE DESCRIPTION**

**Patent documents cited in the description**

- JP 2014180578 A
- JP 2022210208 A